# EUROPEAN PATENT APPLICATION

(11) **EP 4 285 728 A1**
(43) Date of publication of application: **06.12.2023**
(21) Application number: 22176377.4
(22) Date of filing: 31.05.2022
(51) Int. Cl.: A21D 2/26, A21D 8/04, C07K 14/37, C12N 9/42, C12N 9/24, A21D 13/02

(54) **DOUGH COMPOSITION COMPRISING NON-STARCH POLYSACCHARIDE DEGRADING ENZYMES**

(71) Applicant: Kerry Group Services International Limited, Tralee Co. Kerry (IE)
(72) Inventor: Gebhardt, Mathias, Naas (IE); Georis, Jacques, Naas (IE); Kozyr, Oleksandr, Naas (IE); Hanbidge, Alice, Naas (IE); Carr, Derek, Naas (IE); Piarali, Sheila, Naas (IE); Higgins, Niall, Naas (IE); Marshall, Nathan, Naas (IE)
(74) Representative: FRKelly

(57) **Abstract**

The present invention relates to a dough composition comprising flour and at least one enzyme selected from a cellulase enzyme, a beta-glucanase enzyme and a xylanase enzyme; and to the use thereof in baked food products. There is also provided a method of preparing a baked food product, a method of preparing a baked food product having at least one improved property.

## Description

### Field of the Invention

The present invention relates to a dough composition comprising flour and at least one enzyme selected from a cellulase enzyme, a beta-glucanase enzyme and a xylanase enzyme; and to the use thereof in baked food products. There is also provided a method of preparing a baked food product, a method of preparing a baked food product having at least one improved property.

### Background to the Invention

Wheat and rye flour are commonly used in bread manufacturing. Besides protein, lipids and starch, it consists of non-starch polysaccharides (NSP). They play an important role for dough rheology due to their high water-binding capacity and their interaction with gluten.

Typically, rye flour possesses a higher content of NSP compared to wheat flour. With an increasing content of rye flour in the recipe, the impact of NSP on dough handling properties may become more apparent. Dough characteristics and final product quality can benefit by using enzyme, which are able to hydrolyze NPS. It has been reported that dough handling, bread volume, softness and crumb characteristics can be improved. However, an overdosing may lead to a sticky dough causing difficulties in the manufacturing process and can have a detrimental impact on final product quality. It is therefore crucial to apply the correct dosage of a tailored enzyme composition to ensure a consistent bread quality and avoid downtimes in the production process.

Enzymes are proteins, which consists of a sequence of amino acids in its primary structure. They can be produced by fermentation of a bacterial (i.e. *Bacillus*), yeast (i.e. *Saccharomyces*) or fungal (i.e. *Aspergillus*) microorganism or by extraction (i.e. plant). The information about the amino acid sequence is used to classify enzymes into different families (i.e. hydrolases or transferases). It also determines the structure of the active site, a small part of the enzyme, where the biochemical reaction takes place. It plays a crucial role in giving every enzyme its unique properties such as their substrate specificities and their activity at a certain pH or Temperature. Other factors, such as media composition, species of the production organism or process conditions in the fermentation process, also contribute to the type of enzymes overexpressed. Examples of NSP (non-starch polysaccharides) degrading enzymes are cellulases, xylanases or glucanases.

Some enzymes may be produced by using a genetically modified microorganism. They are designed to possess a high degree of purity (less side activities) and an optimized catalytic activity in application. However, many customers prefer an enzyme solution not produced by a recombinant organism. In an organic food production, their usage is prohibited by EU regulation (EU) 2018/848. The application of a "natural" enzyme preparation, not produced by genetically modified microorganism, may be more sophisticated and require a precise application. The mechanism of these enzymes is complex and their performance in application is difficult to predict. It is therefore necessary to characterize their properties *in vitro* and test their performance in bake tests.

The object of this invention is to test the performance of NSP degrading enzymes in improving rye bread quality while maintaining a good processability in the production process. A single enzyme preparation, which has been produced by Trichoderma *reesei, Aspergillus niger* or *Rasamsonia composticola,* has been used. In order to optimize their technical performance and investigate a synergy effect, those enzyme preparations have been combined to a multi-component enzymes preparation.

### Summary of the Invention

According to a first aspect of the present invention, there is provided a dough composition comprising:
(a) flour; and
(b) at least one enzyme selected from a cellulase enzyme, a beta-glucanase enzyme and a xylanase enzyme.

According to a second aspect of the present invention; there is provided a method of preparing a baked food product; the method comprising:
(a) preparing a dough comprising at least one enzyme selected from a cellulase enzyme, a beta-glucanase enzyme and a xylanase enzyme; and
(b) baking the dough for a time and temperature sufficient to yield the bakery product.

According to a third aspect of the present invention; there is provided a method of preparing a baked food product having at least one improved property; the method comprising:
(a) preparing a dough comprising at least one enzyme selected from a cellulase enzyme, a beta-glucanase enzyme and a xylanase enzyme; and
(b) baking the dough for a time and a temperature sufficient to yield the bakery product.

According to a fourth aspect of the present invention, there is provided use of the dough composition in baked foods.

Optionally, the flour is made from any grain suitable for being milled to form flour.

Optionally, the flour comprises wheat flour. Optionally, the flour comprises barley flour. Optionally, the flour comprises oat flour. Optionally, the flour comprises rice flour. Optionally, the flour comprises corn flour. Optionally, the flour comprises wholegrain flour. Optionally, the flour comprises wheat wholegrain flour. Optionally, the flour comprises spelt flour. Optionally, the flour comprises white flour. Optionally, the flour comprises wholemeal flour.

Preferably, the flour comprises rye flour.

Optionally, the flour comprises at least 10% rye flour. Optionally, the flour comprises at least 20% rye flour. Optionally, the flour comprises at least 30% rye flour. Optionally, the flour comprises at least 40% rye flour. Optionally, the flour comprises at least 50% rye flour. Optionally, the flour comprises at least 60% rye flour. Optionally, the flour comprises at least 70% rye flour. Optionally, the flour comprises at least 80% rye flour. Optionally, the flour comprises at least 90% rye flour. Optionally, the flour comprises 100% rye flour.

Optionally, the flour comprises at least 20% wheat flour. Optionally, the flour comprises at least 30% wheat flour. Optionally, the flour comprises at least 40% wheat flour. Optionally, the flour comprises at least 50% wheat flour. Optionally, the flour comprises at least 60% wheat flour. Optionally, the flour comprises at least 70% wheat flour. Optionally, the flour comprises at least 80% wheat flour. Optionally, the flour comprises at least 90% wheat flour.

Optionally, the flour comprises at least 40% wholemeal flour. Optionally, the flour comprises at least 50% wholemeal flour. Optionally, the flour comprises at least 60% wholemeal flour. Optionally, the flour comprises at least 70% wholemeal flour. Optionally, the flour comprises at least 80% wholemeal flour.

Preferably, the flour comprises 70% rye flour. Further preferably, the flour comprises 70% rye flour and 30% wheat flour.

Alternatively, the flour comprises 40% wheat flour. Further alternatively, the flour comprises 40% wheat flour and 60% wholemeal flour.

Optionally, the cellulase enzyme is beta-1,4-endoglucanase. Further preferably, the cellulase enzyme is endo-(1-4)-beta-D-glucanase.

Optionally, the cellulase enzyme is of plant origin. Optionally, the cellulase enzyme is of bacterial origin.

Preferably, the cellulase enzyme is of fungal origin.

Optionally, the cellulase activity comprises at least 50% of the total activity of the enzyme. Optionally, the cellulase activity comprises at least 60% of the total activity of the enzyme. Optionally, the cellulase activity comprises at least 70% of the total activity of the enzyme. Optionally, the cellulase activity comprises at least 80% of the total activity of the enzyme. Optionally, the cellulase activity comprises at least 90% of the total activity of the enzyme. Optionally, the cellulase activity comprises at least 95% of the total activity of the enzyme. Optionally, the cellulase activity comprises 100% of the total activity of the enzyme.

Optionally, the cellulase enzyme belongs to the glycoside hydrolases (GH) of family 5; and/or the glycoside hydrolases of family 7; and/or the glycoside hydrolases of family 12; and/or the glycoside hydrolases of family 45.

Optionally, the cellulase enzyme is derived from any one or more of the genera *Trichoderma, Rasamsonia* and *Aspergillus.*

Optionally, the cellulase enzyme is present in an amount of 10 to 5000 units/kg flour. Optionally, the cellulase enzyme is present in an amount of 12 to 4500 units/kg flour. Optionally, the cellulase enzyme is present in an amount of 14 to 4000 units/kg flour. Optionally, the cellulase enzyme is present in an amount of 16 to 3500 units/kg flour. Optionally, the cellulase enzyme is present in an amount of 18 to 3000 units/kg flour. Optionally, the cellulase enzyme is present in an amount of 20 to 2900 units/kg flour. Optionally, the cellulase enzyme is present in an amount of 22 to 2800 units/kg flour. Optionally, the cellulase enzyme is present in an amount of 24 to 2750 units/kg flour.

Preferably, the cellulase enzyme is present in an amount of 29 to 2707 units/kg flour.

Optionally, the cellulase enzyme has at least 70% identity to one or more of SEQ ID NO:1 to SEQ ID NO:49. Optionally, the cellulase enzyme has at least 80% identity to one or more of SEQ ID NO:1 to SEQ ID NO:49. Optionally, the cellulase enzyme has at least 85% identity to one or more of SEQ ID NO:1 to SEQ ID NO:49. Optionally, the cellulase enzyme has at least 90% identity to one or more of SEQ ID NO:1 to SEQ ID NO:49. Optionally, the cellulase enzyme has at least 95% identity to one or more of SEQ ID NO:1 to SEQ ID NO:49. Optionally, the cellulase enzyme has at least 98% identity to one or more of SEQ ID NO:1 to SEQ ID NO:49.

Preferably, the cellulase enzyme is selected from any one or more of SEQ ID NO:1 to SEQ ID NO:49.

Preferably, the cellulase enzyme is derived from the fungus of the genus *Trichoderma.*

Further preferably, the cellulase enzyme is derived from the fungus of the species *Trichoderma reesei.*

Optionally, the cellulase enzyme is selected from any one of more of SEQ ID NO:5 to SEQ ID NO:8.

Preferably, the cellulase enzyme is selected from SEQ ID NO:5; and/or SEQ ID NO:6; and/or SEQ ID NO:7; and/or SEQ ID NO:8;

Alternatively, the cellulase enzyme belongs to the glycoside hydrolases (GH) of family 5; and/or the glycoside hydrolases of family 6; and/or the glycoside hydrolases of family 7; and/or the glycoside hydrolases of family 12; and/or the glycoside hydrolases of family 45.

Optionally, the cellulase enzyme is derived from the fungus of the genus *Aspergillus.*

Further optionally, the cellulase enzyme is derived from the fungus of the species *Aspergillus niger.*

Optionally, the cellulase enzyme is selected from any one of more of SEQ ID NO:15 to SEQ ID NO:24 and SEQ ID NO:45 to SEQ ID NO:49.

Further optionally, the cellulase enzyme is selected from of SEQ ID NO:15; and/or SEQ ID NO:16; and/or SEQ ID NO:24; and/or SEQ ID NO:45; and/or SEQ ID NO:46; and/or SEQ ID NO:47; and/or SEQ ID NO:48; and/or SEQ ID NO:49.

Optionally, the cellulase enzyme is derived from the fungus of the genus *Rasamsonia.*

Further optionally, the cellulase enzyme is derived from the fungus of the species *Rasamsonia composticola.*

Optionally, the cellulase enzyme is selected from any one of more of SEQ ID NO:25 to SEQ ID NO:33.

Optionally, the cellulase enzyme is selected from of SEQ ID NO:25; and/or SEQ ID NO:26; and/or SEQ ID NO:28; and/or SEQ ID NO:29; and/or SEQ ID NO:30; and/or SEQ ID NO:31; and/or SEQ ID NO:32; and/or SEQ ID NO:33.

Preferably, the beta-glucanase enzyme is endo-1,3:1,4-beta-glucanase.

Optionally, the beta-glucanase enzyme is of plant origin. Optionally, the beta-glucanase enzyme is of bacterial origin.

Preferably, the beta-glucanase enzyme is of fungal origin.

Optionally, the beta-glucanase activity comprises at least 50% of the total activity of the enzyme. Optionally, the beta-glucanase activity comprises at least 60% of the total activity of the enzyme. Optionally, the beta-glucanase activity comprises at least 70% of the total activity of the enzyme. Optionally, the beta-glucanase activity comprises at least 80% of the total activity of the enzyme. Optionally, the beta-glucanase activity comprises at least 90% of the total activity of the enzyme. Optionally, the beta-glucanase activity comprises at least 95% of the total activity of the enzyme. Optionally, the beta-glucanase activity comprises 100% of the total activity of the enzyme.

Optionally, the beta-glucanase enzyme belongs to the glycoside hydrolases (GH) of family 5; and/or the glycoside hydrolases of family 7; and/or the glycoside hydrolases of family 12; and/or the glycoside hydrolases of family 16; and/or the glycoside hydrolases of family 17; and/or the glycoside hydrolases of family 45.

Optionally, the beta-glucanase enzyme is derived from any one or more of the genera *Trichoderma, Rasamsonia* and *Aspergillus.*

Optionally, the beta-glucanase enzyme is present in an amount of 10 to 19400 units/kg flour. Optionally, the beta-glucanase enzyme is present in an amount of 14 to 18495 units/kg flour. Optionally, the beta-glucanase enzyme is present in an amount of 18 to 17590 units/kg flour. Optionally, the beta-glucanase enzyme is present in an amount of 22 to 16685 units/kg flour. Optionally, the beta-glucanase enzyme is present in an amount of 26 to 15780 units/kg flour. Optionally, the beta-glucanase enzyme is present in an amount of 30 to 14875 units/kg flour. Optionally, the beta-glucanase enzyme is present in an amount of 34 to 13970 units/kg flour. Optionally, the beta-glucanase enzyme is present in an amount of 35 to 13065 units/kg flour. Optionally, the beta-glucanase enzyme is present in an amount of 36 to 12160 units/kg flour. Optionally, the beta-glucanase enzyme is present in an amount of 37 to 11255 units/kg flour.

Preferably, the beta-glucanase enzyme is present in an amount of 37 to 11251 units/kg flour.

Optionally, the beta-glucanase enzyme has at least 70% identity to any one of more of SEQ ID NO:1 to SEQ ID NO:49. Optionally, the beta-glucanase enzyme has at least 75% identity to any one of more of SEQ ID NO:1 to SEQ ID NO:49. Optionally, the beta-glucanase enzyme has at least 80% identity to any one of more of SEQ ID NO:1 to SEQ ID NO:49. Optionally, the beta-glucanase enzyme has at least 85% identity to any one of more of SEQ ID NO:1 to SEQ ID NO:49. Optionally, the beta-glucanase enzyme has at least 90% identity to any one of more of SEQ ID NO:1 to SEQ ID NO:49. Optionally, the beta-glucanase enzyme has at least 95% identity to any one of more of SEQ ID NO:1 to SEQ ID NO:49. Optionally, the beta-glucanase enzyme has at least 98% identity to any one of more of SEQ ID NO:1 to SEQ ID NO:49.

Preferably, the beta-glucanase enzyme is selected from any one of more of SEQ ID NO:1 to SEQ ID NO:49.

Optionally, the beta-glucanase enzyme is derived from the fungus of the genus *Trichoderma.*

Optionally, the beta-glucanase enzyme is derived from the fungus of the species *Trichoderma reesei.*

Optionally, the beta-glucanase enzyme is selected from any one of more of SEQ ID NO:5 to SEQ ID NO:8.

Preferably, the beta-glucanase enzyme is selected from SEQ ID NO:5; and/or SEQ ID NO:6; and/or SEQ ID NO:7; and/or SEQ ID NO:8

Optionally, the beta-glucanase enzyme is derived from the fungus of the genus *Aspergillus.*

Optionally, the beta-glucanase enzyme is derived from the fungus of the species *Aspergillus niger.*

Optionally, the beta-glucanase enzyme is selected from any one of more of SEQ ID NO:15 to SEQ ID NO:24 and SEQ ID NO:39 to SEQ ID NO:49.

Further optionally, the beta-glucanase enzyme is selected from of SEQ ID NO:15; and/or SEQ ID NO:16; and/or SEQ ID NO:19; and/or SEQ ID NO:20; and/or SEQ ID NO:21; and/or SEQ ID NO:22; and/or SEQ ID NO:24; and/or SEQ ID NO:39; and/or SEQ ID NO:40 and/or SEQ ID NO:41; and/or SEQ ID NO:44; and/or SEQ ID NO:45; and/or SEQ ID NO:46; and/or SEQ ID NO:47; and/or SEQ ID NO:48; and/or SEQ ID NO:49.

Preferably, the beta-glucanase enzyme is derived from the fungus of the genus *Rasamsonia.*

Further preferably, the beta-glucanase enzyme is derived from the fungus of the species *Rasamsonia composticola.*

Optionally, the beta-glucanase enzyme is selected from any one of more of SEQ ID NO:25 to SEQ ID NO:33.

Preferably, the beta-glucanase enzyme is selected from of SEQ ID NO:25; and/or SEQ ID NO:26; and/or SEQ ID NO:28; and/or SEQ ID NO:29; and/or SEQ ID NO:30; and/or SEQ ID NO:31; and/or SEQ ID NO:32; and/or SEQ ID NO:33.

Preferably, the xylanase enzyme is endo-1,4-beta-D-xylanase. Further preferably, the xylanase enzyme is a GH10 xylanase enzyme and/or a GH11 xylanase enzyme.

Optionally, the xylanase enzyme is of plant origin. Optionally, the xylanase enzyme is of bacterial origin.

Preferably, the xylanase enzyme is of fungal origin.

Optionally, the xylanase activity comprises at least 50% of the total activity of the enzyme. Optionally, the xylanase activity comprises at least 60% of the total activity of the enzyme. Optionally, the xylanase activity comprises at least 70% of the total activity of the enzyme. Optionally, the xylanase activity comprises at least 80% of the total activity of the enzyme. Optionally, the xylanase activity comprises at least 90% of the total activity of the enzyme. Optionally, the xylanase activity comprises at least 95% of the total activity of the enzyme. Optionally, the xylanase activity comprises 100% of the total activity of the enzyme.

Optionally, the xylanase enzyme belongs to the glycoside hydrolases (GH) of family 11; and/or the glycoside hydrolases of family 10.

Optionally, the xylanase enzyme is derived from any one or more of the genera *Trichoderma, Rasamsonia* and *Aspergillus.*

Optionally, the xylanase enzyme is present in an amount of 10 to 900 units/kg flour. Optionally, the xylanase enzyme is present in an amount of 12 to 736 units/kg flour. Optionally, the xylanase enzyme is present in an amount of 14 to 572 units/kg flour. Optionally, the xylanase enzyme is present in an amount of 16 to 408 units/kg flour. Optionally, the xylanase enzyme is present in an amount of 18 to 244 units/kg flour.

Preferably, the xylanase enzyme is present in an amount of 18 to 243 units/kg flour.

Optionally, the xylanase enzyme has at least 70% identity to any one of more of SEQ ID NO:1 to SEQ ID NO:49. Optionally, the xylanase enzyme has at least 75% identity to any one of more of SEQ ID NO:1 to SEQ ID NO:49. Optionally, the xylanase enzyme has at least 80% identity to any one of more of SEQ ID NO:1 to SEQ ID NO:49. Optionally, the xylanase enzyme has at least 85% identity to any one of more of SEQ ID NO:1 to SEQ ID NO:49. Optionally, the xylanase enzyme has at least 90% identity to any one of more of SEQ ID NO:1 to SEQ ID NO:49. Optionally, the xylanase enzyme has at least 95% identity to any one of more of SEQ ID NO:1 to SEQ ID NO:49. Optionally, the xylanase enzyme has at least 98% identity to any one of more of SEQ ID NO:1 to SEQ ID NO:49.

Preferably, the xylanase enzyme is selected from any one of more of SEQ ID NO:1 to SEQ ID NO:49.

Optionally, the xylanase enzyme is derived from the fungus of the genus *Trichoderma.*

Optionally, the xylanase enzyme is derived from the fungus of the species *Trichoderma reesei.*

Optionally, the xylanase enzyme is selected from any one of more of SEQ ID NO:1 to SEQ ID NO:4.

Optionally, xylanase enzyme is selected from SEQ ID NO:1; and/or SEQ ID NO:2; and/or SEQ ID NO:3; and/or SEQ ID NO:4.

Optionally, the xylanase enzyme is derived from the fungus of the genus *Rasamsonia.*

Optionally, the xylanase enzyme is derived from the fungus of the species *Rasamsonia composticola.*

Optionally, the xylanase enzyme is selected from SEQ ID NO:27

Preferably, the xylanase enzyme is derived from the fungus of the genus *Aspergillus.*

Further preferably, the xylanase enzyme is derived from the fungus of the species *Aspergillus niger.*

Optionally, the xylanase enzyme is selected from any one of more of SEQ ID NO:10 to SEQ ID NO:14 and SEQ ID NO:34 to SEQ ID NO:38.

Preferably, the xylanase enzyme is selected from of SEQ ID NO:10; and/or SEQ ID NO:11; and/or SEQ ID NO:12; and/or SEQ ID NO:13; and/or SEQ ID NO:14; and/or SEQ ID NO:34; and/or SEQ ID NO:35; and/or SEQ ID NO:36; and/or SEQ ID NO:37; and/or SEQ ID NO:38.

Optionally, the dough composition comprises a cellulase enzyme.

Optionally, the dough composition comprises a xylanase enzyme.

Optionally, the dough composition comprises a beta-glucanase enzyme.

Optionally, the dough composition comprises a cellulase enzyme and a xylanase enzyme.

Optionally, the dough composition comprises a cellulase enzyme and a beta-glucanase enzyme.

Optionally, the dough composition comprises a xylanase enzyme and a beta-glucanase enzyme.

Preferably, the dough composition comprises a cellulase enzyme, a xylanase enzyme and a beta-glucanase enzyme.

Further preferably, the dough composition comprises a cellulase enzyme derived from the fungus of the genus *Trichoderma,* or a cellulase enzyme derived from the fungus of the genus *Aspergillus;* and a xylanase enzyme derived from the fungus of the genus *Aspergillus;* and a beta-glucanase enzyme derived from the fungus of the genus *Rasamsonia.*

Even further preferably, the dough composition comprises a cellulase enzyme derived from the fungus of the species *Trichoderma reesei,* or a cellulase enzyme derived from the fungus of the species *Aspergillus niger*; and a xylanase enzyme derived from the fungus of the species *Aspergillus niger;* and a beta-glucanase enzyme derived from the fungus of the species *Rasamsonia composticola.*

Optionally, the dough composition may comprise further ingredients, such as water, milk powder, sugar, gluten and soy, eggs yeast, salt, eggs, gluten, sourdough in the from dried or liquid or stiff, ascorbic acid, shortening, other enzymes, hydrocolloid, fibres, proteins, seed, other cereals, fruits, other edible inclusions, and mixtures thereof.

Optionally, the baked food product any kind of baked food, including but not limited to bread, bread rolls and cakes.

Optionally, in the method of preparing a baked food product, the dough is the prepared and the baking is performed in any suitable manner for dough production and/or baked product production.

Optionally, the method of preparing a baked food product comprises:
(a) preparing a dough comprising at least one enzyme selected from a cellulase enzyme, a beta-glucanase enzyme and a xylanase enzyme by mixing the dough composition for between 2-20 mins; and
(b) baking the dough for a time and temperature sufficient to yield the bakery product.

Optionally, the method of preparing a baked food product comprises:
(a) preparing a dough comprising at least one enzyme selected from a cellulase enzyme, a beta-glucanase enzyme and a xylanase enzyme by mixing the dough composition for between 2-20 mins;
(b) leaving the dough composition to rest for between 2-240 mins; and
(c) baking the dough for a time and temperature sufficient to yield the bakery product.

Optionally, the method of preparing a baked food product comprises:
(a) preparing a dough comprising at least one enzyme selected from a cellulase enzyme, a beta-glucanase enzyme and a xylanase enzyme by mixing the dough composition for between 2-20 mins;
(b) leaving the dough composition to rest for between 2-240 mins;
(c) leaving the dough composition to prove for between 10-240 mins at 20°C-45°C and 70-90% relative humidity; and
(d) baking the dough for a time and temperature sufficient to yield the bakery product.

Optionally, the method of preparing a baked food product comprises:
(a) preparing a dough comprising at least one enzyme selected from a cellulase enzyme, a beta-glucanase enzyme and a xylanase enzyme by mixing the dough composition for between 2-20 mins;
(b) leaving the dough composition to rest for between 2-240 mins;
(c) leaving the dough composition to prove for between 10-90 mins at 20°C-45°C and 70-90% relative humidity; and
(d) baking the dough for 10-180 mins at 150-300°C.

Optionally, the method of preparing a baked food product comprises:
(a) preparing a dough comprising at least one enzyme selected from a cellulase enzyme, a beta-glucanase enzyme and a xylanase enzyme by mixing the dough composition with 1000 turns/revolutions (slow);
(b) leaving the dough composition to rest for 20 mins;
(c) leaving the dough composition to prove for 45 mins at 38°C and 80% relative humidity; and
(d) baking the dough for 1 min at 260°C (top temperature) and 250°C (bottom temperature), followed by 59 mins at 200°C (top and bottom temperature).

Optionally, the method of preparing a baked food product comprises:
(a) preparing a dough comprising at least one enzyme selected from a cellulase enzyme, a beta-glucanase enzyme and a xylanase enzyme by mixing the dough composition with 400 turns/revolutions (slow) and 1800 turns/revolutions (fast);
(b) leaving the dough composition to rest for 10 mins and further 20min (after moulding);
(c) leaving the dough composition to prove for 60 mins at 32°C and 80% relative humidity; and
(d) baking the dough for 45min 230°C (top temperature) and 260 °C (bottom temperature).

Optionally, the method of preparing a baked food product comprises:
(a) preparing a dough comprising at least one enzyme selected from a cellulase enzyme, a beta-glucanase enzyme and a xylanase enzyme by mixing the dough composition with 500 turns/revolutions (slow) and 1300 turns/revolutions (fast);
(b) leaving the dough composition to rest for 10 mins and further 10min (after moulding);
(c) leaving the dough composition to prove for 40 mins at 38°C and 85% relative humidity; and
(d) baking the dough for 35 min at 235°C (top temperature) and 270°C (bottom temperature).

Optionally, in the method of preparing a baked food product having at least one improved property, wherein the improved property is selected from any one or more volume, softness, butterability and balling after slicing.

Optionally, in the method of preparing a baked food product having at least one improved property, wherein the improved property is selected from any one or more of specific loaf volume, improved loaf volume, specific volume, improved bread volume, softness day up to day +7 post baking, butterability, softness to touch, balling after slicing, softness to touch and softness to bite.

Optionally, the method of preparing a baked food product having at least one improved property further comprises maintaining at least one property.

Optionally, the method of preparing a baked food product having at least one improved property further comprises maintaining at least one property, wherein the maintained properties are selected from any one of more of processability, height:witdh ratio, sliceability, sensory (flavour profile), slicing / balling, crumb colour, crumb resilience, aroma, cohesiveness and moistness.

Optionally, in the method of preparing a baked food product having at least one improved property, the improved property is selected from any one or more of specific loaf volume, improved loaf volume, specific volume, improved bread volume, softness day+1, softness day+5, butterability, softness to touch, balling after slicing, softness to touch and softness to bite; whilst maintaining any one of more of processability, height:witdh ratio, sliceability, sensory (flavour profile), slicing / balling, crumb colour, crumb resilience, aroma, cohesiveness and moistness.

Optionally, the improved property may be determined by comparing the baked food product prepared with and without the dough comprising at least one enzyme selected from a cellulase enzyme, a beta-glucanase enzyme and a xylanase enzyme.

Optionally, in the use of the dough composition in baked foods; the baked foods can be any kind of baked foods, including but not limited to bread, bread rolls and cakes.

### Brief Description of the Drawings

The invention will be described with reference to the accompanying drawings in which:
**Figure 1** illustrates the experimental design of bake tests;
**Figure 2** illustrates temperature and pH profiles of (A/B) cellulase (R. *reesei*); (C/D) xylanase (A. *Niger*); (E/F) beta-glucanase (R. *composticola*);
**Figure** 3 illustrates pH and temperature profile of two xylanases (A); cellulase (B) as part of a validation test of example 7;
**Figure 4** illustrates cellulase impact on improved loaf volume;
**Figure 5** illustrates xylanase impact on improved loaf volume;
**Figure 6** illustrates beta-glucanase impact on improved loaf volume;

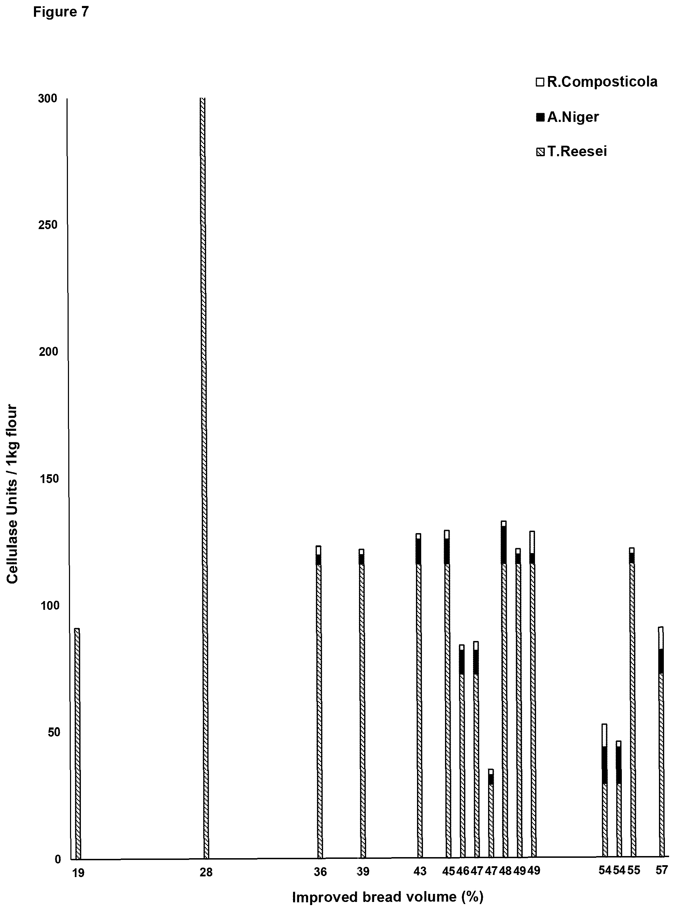

**Figure 7** illustrates cellulase impact on improved softness;

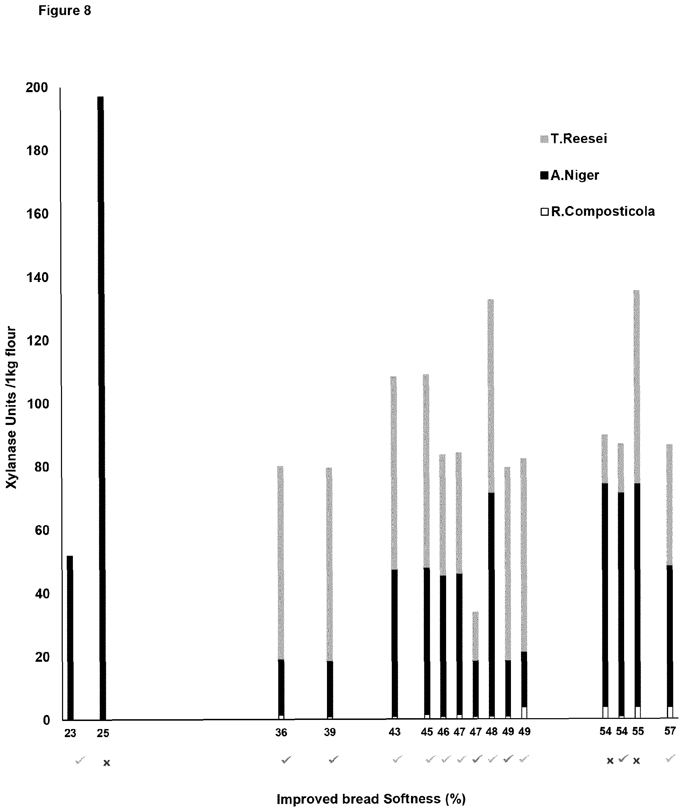

**Figure 8** illustrates xylanase impact on improved softness;

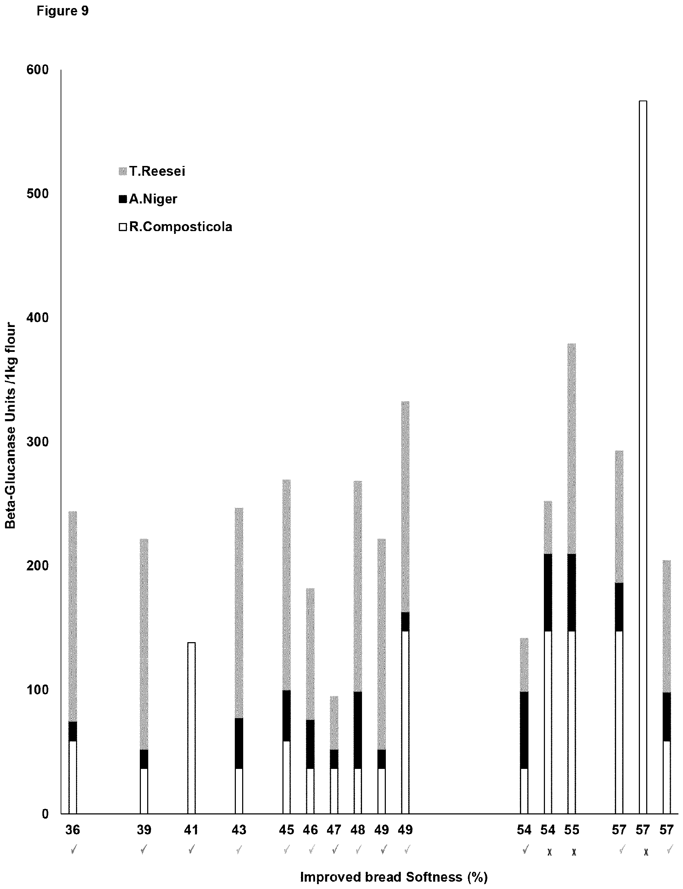

**Figure 9** illustrates beta-glucanase impact on improved softness;
**Figure 10** illustrates correlation of an improved volume between breads proven in a tin (loaf) and a basket;
**Figure 11** illustrates correlation of improves softness between 24hrs post baking and day 5 in loaf breads;
**Figure 12** illustrates the effect of a single enzyme preparation from *Trichoderma reesei* (cellulase) on rye bread loafs;
**Figure 13** illustrates the effect of a single enzyme preparation from *Trichoderma reesei* (cellulase) on rye bread (basked)
**Figure 14** illustrates the effect of a single enzyme preparation from *Aspergillus niger* (xylanase) on rye bread (loaf);
**Figure 15** illustrates the effect of a single enzyme preparation from *Aspergillus niger* (xylanase) on rye bread (basked)
**Figure 16** illustrates the effect of a single enzyme preparation from *Rasamsonia composticola* (beta-glucanase) on rye bread (loaf);
**Figure 17** illustrates the effect of a single enzyme preparation from *Rasamsonia composticola* (beta-glucanase) on rye bread (basked)
**Figure 18** illustrates the effect of a multi-component enzyme preparation on loaf volume;
**Figure 19** illustrates the effect of a multi-component enzyme preparation on bread volume (basket);
**Figure 20** illustrates the effect of a multi-component enzyme preparation on loaf volume;
**Figure 21** illustrates the effect of a multi-component enzyme preparation on bread volume (basket);
**Figure 22** illustrates the effect of a multi-component enzyme preparation on loaf volume;
**Figure 23** illustrates the effect of a multi-component enzyme preparation on bread volume (basket);

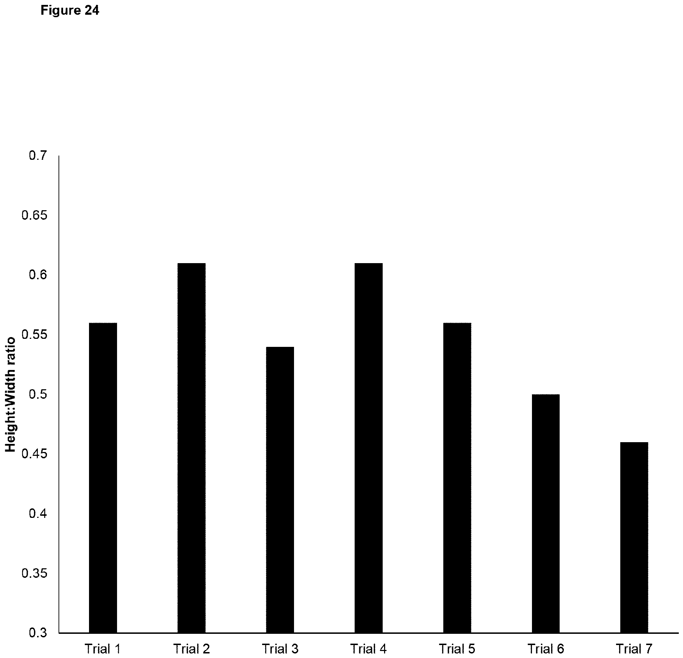

**Figure 24** illustrates Height:Width ratio of a commercial product vs MCP (Multi-component enzyme preparation);
**Figure 25** illustrates improved loaf volume (%) compared to control;
**Figure 26** illustrates a commercial product vs MCP (Day+1 and Day+4) -improved softness compared to Control;
**Figure 27** illustrates dose response of a commercial product against negative control (no enzymes);
**Figure 28** illustrates dose response of MCP against negative control (no enzymes); and
**Figure 29** illustrates range of total and main enzyme activity applied a SEP and MCP, which showed promising performance in bake trials

### Examples

The invention will now be described with reference to the following non-limiting examples.

### Abbreviations

NSP - Non-starch polysaccharides
MCP - Multi component preparation
SEP - Single enzyme preparation
Cell - Cellulase
Xyl - Xylanase
BG - Beta Glucanase

### Materials & Methods

### Example 1

### In Vitro Analysis

A cellulase particularly preferred for use according to the present invention is an endo-(1-4)-beta-D-glucanase (activity of which is evidenced through the hydrolysis of azurine-crosslinked HE-cellulose for the purpose of this study) having the following properties:
**Substrate specificity:** To quantify the Cellulase activity, an AZCL-HE-Cellulose substrate was used. The substrate was prepared by dyeing and cross-linking HE-cellulose to produce a material which hydrates in water but is water insoluble. Hydrolysis by endo-(1-4)-beta-D-glucanase (cellulase) produces water soluble dyed fragments and the rate of release of these (increase in absorbance at 590 nm) can be related directly to enzyme activity.

**Activity:** The standard conditions of the assay were pH 4.5 with a temperature of 40°C. An aqueous suspension containing 100mM sodium acetate (pH 4.5) was used to suitably dilute the enzyme. The substrate (in tablet form) was added to the cellulase and incubated for 10 minutes at 40°C. In order to characterise cellulase temperature & pH optima, a temperature and pH response curve was conducted. A pH curve was defined by the relationship between enzymatic activity and relative pH-that ranges between minima at pH 2 and maximum at pH 9. A temperature curve was similarly defined as the relationship between enzymatic activity and relative temperature - that ranges between minima at 30°C to maximum at 80°C.

**Unit of measurement:** One Unit of activity is defined as the amount of enzyme required to release one micromole of glucose reducing-sugar-equivalents per minute from either CM-cellulose 4M (10 mg/mL; Lot 81101) at pH 4.5 and 40°C.

A Xylanase particularly preferred for use according to the present invention is an endo-1,4-beta-D-xylanase having the following properties:
**Substrate specificity:** The substrate employed is Azurine-crosslinked wheat arabinoxylan. This substrate is prepared by dyeing and crosslinking highly purified wheat arabinoxylan to produce a material which hydrates in water but is water insoluble. Hydrolysis by endo-1,4-beta-D-xylanase produces water soluble dyed fragments, and the rate of release of these (increase in absorbance at 590 nm) can be related directly to enzyme activity.

**Activity:** The standard conditions of the assay were pH 4.7 with a temperature of 40°C. An aqueous suspension containing 100mM sodium acetate (pH 4.5) was used to suitably dilute the enzyme. The substrate (in tablet form) was added to the Xylanase and incubated for 10 minutes at 40°C. A pH curve was defined by the relationship between enzymatic activity and relative pH-that ranges between minima at pH 2 and maximum at pH 9. A temperature curve was similarly defined as the relationship between enzymatic activity and relative temperature - that ranges between minima at 30°C to maximum at 70°C.

**Unit of measurement:** One Unit of enzyme activity is the amount of enzyme required to release one micromole of reducing sugar equivalents (as xylose by the Nelson-Somogyi reducing-sugar method) from arabinoxylan per minute under standard assay conditions (40°C and pH 4.7).

A beta-glucanase particularly preferred for use according to the present invention is an endo-1,3:1,4-beta-glucanase (activity of which is evidenced through the hydrolysis of Azurine-crosslinked barley beta-glucan for the purpose of this study) having the following properties:
**Substrate specificity:** The substrate employed is Azurine-crosslinked barley beta-glucan (AZCL-Beta-Glucan). The substrate is prepared by dyeing and cross linking highly purified beta-glucan to produce a material which hydrates in water but is water insoluble. Hydrolysis by malt beta-glucanase or bacterial 1,3:1,4-beta-glucanase, produces water soluble dyed fragments, and the rate of release of these (increase in absorbance at 590 nm) can be related directly to enzyme activity.

**Activity:** The standard conditions of the assay were pH 4.5 with a temperature of 40°C. An aqueous suspension containing 100mM sodium acetate (pH 4.5) was used to suitably dilute the enzyme. The substrate (in tablet form) was added to the beta-glucanase and incubated for 10 minutes at 40°C.In order to characterise beta-glucanase temperature & pH optima, a temperature and pH response curve was conducted. A pH curve was defined by the relationship between enzymatic activity and relative pH-that ranges between minima at pH 2 and maximum at pH 9. A temperature curve was similarly defined as the relationship between enzymatic activity and relative temperature - that ranges between minima at 30°C to maximum at 80°C.

**Unit of measurement:** One Unit of activity is defined as the amount of enzyme required to release one micromole of reducing-sugar equivalents per minute under the defined assay conditions. **Substrate specificity:** The substrate is a barely β-Glucan powder. Betaglucanases hydrolyse beta-D-glucans to yield a variety of oligosaccharides. The reducing sugar groups released are reacted with 3.5 Dinitro Salicylic Acid (DNS). The colour change produced is proportional to the amount of reducing sugars released. This in turn is proportional to the activity of the betaglucanase present in the sample. The optical density is read at 540nm and converted into mg of reducing sugar produced (expressed as maltose) using a standard curve.

**Activity:** The standard conditions of the assay were pH 5 with a temperature of 50°C. An aqueous suspension containing 1M acetate buffer (pH 5) was used to suitably dilute the enzyme. The substrate (once dissolved) was added to the beta-glucanase and incubated for 10 minutes at 50°C.

**Unit of measurement:** The colour change observed in the assay is proportional to the amount of reducing sugars released. This in turn is proportional to the activity of the betaglucanase present in the sample. The optical density is read at 540nm and converted into mgs. of reducing sugar produced (expressed as maltose) using a standard curve.

### Example 2

**Sequencing**

| **Name** | **GH Family** | **Sequence** |
|---|---|---|
| SEQ ID NO: 1 - *Trichoderma reesei* | 11 | MVSFTSLLAGVAAISGVLAAPAAEVESVAVEKRQTIQP GTGYNNGYFYSYWNDGHGGVTYTNGPGGQFSVNW SNSGNFVGGKGWQPGTKNKVINFSGSYNPNGNSYLS VYGWSRNPLIEYYIVENFGTYNPSTGATKLGEVTSDGS |
| | | VYDIYRTQRVNQPSIIGTATFYQYWSVRRNHRSSGSVN TANHFNAWAQQGLTLGTMDYQIVAVEGYFSSGSASITVS |
| SEQ ID NO: 2 - *Trichoderma reesei* | 10 | MKANVILCLLAPLVAALPTETIHLDPELAALRANLTERTAD LWDRQASQSIDQLIKRKGKLYFGTATDRGLLQREKNAAII QADLGQVTPENSMKWQSLENNQGQLNWGDADYLVN FAQQNGKSIRGHTLIWHSQLPAWVNNINNADTLRQVIR THVSTVVGRYKGKIRAWDVVNEIFNEDGTLRSSVFSRLLG EEFVSIAFRAARDADPSARLYINDYNLDRANYGKVNGLKT YVSKWISQGVPIDGIGSQSHLSGGGGSGTLGALQQLATVP VTELAITELDIQGAPTTDYTQVVQACLSVSKCVGITVWGIS DKDSWRASTNPLLFDANFNPKPAYNSIVGILQ |
| SEQ ID NO: 3 - *Trichoderma reesei* | 11 | MVAFSSLICALTSIASTLAMPTGLEPESSVNVTERGMYDFV LGAHNDHRRRASINYDQNYQTGGQVSYSPSNTGFSVNW NTQDDFVVGVGWTTGSSAPINFGGSFSVNSGTGLLSVYG WSTNPLVEYYIMEDNHNYPAQGTVKGTVTSDGATYTIWE NTRVNEPSIQGTATFNQYISVRNSPRTSGTVTVQNHFNAW ASLGLHLGQMNYQVVAVEGWGGSGSASQSVSN |
| SEQ ID NO: 4 - *Trichoderma reesei* | 11 | MGPLEESINANITERGPNNFVLGGHNAVRRAAINYNQDYT TGGDVVYTHSNTGFAVNWSYPNDFVVGVGWNPGGSAPI NFSGNFGVGSGVGLLSVYGWSTNPLVEYYVVEDNFGFSSG GTVKGSVTSDGSSYTIWENTRVNEPSIVGTATFN |
| SEQ ID NO: 5 - *Trichoderma reesei* | 5 | MWVVYPPLKNFTGSNNYPDGIGQMQHFVNDDGMTIFRLPV GWQYLVNNNLGGNLDSTSISKYDQLVQGCLSLGAYCIVDIH NYARWNGGIIGQGGPTNAQFTSLWSQLASKYASQSRVWFG IMNEPHDVNINTWAATVQEVVTAIRNAGATSQFISLPGNDW QSAGAFISDGSAAALSQVTNPDGSTTNLIFDVHKYLDSDNS GTHAECTTNNIDGAFSPLATWLRQNNRQAILTETGGGNVQS CIQDMCQQIQYLNQNSDVYLGYVGWGAGSFDSTEASIPDLT GRVALVTGGNHIGHFVLTTKLLPLLKATASTTGSDVRIVTISS VAEKFAPRHNTFATTKDLSDPGTADPNDYTSRKTVFSRYGA SKLANLLFTRELQRRLDEEDANIMALAVDPGPVATDGGMGV FPGFLKPVLKLVMKSP |
| SEQ ID NO: 6 - *Trichoderma reesei* | 7 | MAPSVTLPLTTAILAIARLVAAQQPGTSTPEVHPKLTTYKCT KSGGCVAQDTSVVLDWNYRWMHDANYNSCTVNGGVNT TLCPDEATCGKNCFIEGVDYAASGVTTSGSSLTMNQYMPS SSGGYSSVSPRLYLLDSDGEYVMLKLNGQELSFDVDLSALP CGENGSLYLSQMDENGGANQYNTAGANYGSGYCDAQCPV QTWRNGTLNTSHQGFCCNEMDILEGNSRANALTPHSCTA TACDSAGCGFNPYGSGYKSYYGPGDTVDTSKTFTIITQFNT DNGSPSGNLVSITRKYQQNGVDIPSAQPGGDTISSCPSASA |
| | | YGGLATMGKALSSGMVLVFSIWNDNSQYMNWLDSGNAGP CSSTEGNPSNILANNPNTHVVFSNIRWGDIGSTTNSTAPPPP PASSTTFSTTRRSSTTSSSPSCTQTHWGQCGGIGYSGCKT CTSGTTCQYSNDYYSQCL |
| SEQ ID NO: 7 - *Trichoderma reesei* | 45 | MVTGATGGLGSQLTRAILECGGDVIAIDAHEIPLATTWEPLK ALAKSLSRTLVYHACDITSPSLLETVFESAASQVKHPLRGLV NCAGIGTAAESINYPESQTRQTLDVNLAGSLYVAQAAAKVV RRQYQEGKSPGASFVFVASMSGYIANKATPTAIYCASKAGV HQLTRSLAAEWGSPSSPLSAFSSSADAAAAAAATAAAGPVI RVNSISPGVIATPMTQQFLGRQDWTSTWQEEAMLKRISTPD EYRGAIAFLLGDASSYVTGTDLRIDGGSTGCFERYYDGQEG ACGCGSSSGAFPWQLGIGNGVYTAAGSQALFDTAGASWC GAGCGKCYQLTSTGQAPCSSCGTGGAAGQSIIVMVTNLCP NNGNAQWCPVVGGTNQYGYSYHFDIMAQNEIFGDNVVVDF EPIACPGQAASDWGTCLCVGQQETDPTPVLGNDTGSTPPG SSPPATSSSPPSGGGQQTLYGQCGGAGWTGPTTCQAPGT CKVQNQWYSQCLP |
| SEQ ID NO: 8 - *Trichoderma reesei* | 5 | MRATSLLAAALAVAGDALAGKIKYLGVAIPGIDFGCDIDGSCP TTSSVPLLSYKGGDGAGQMKHFAEDDGLNVFRISATWQFVL NNTVDGKLDELNWGSYNKVVNACLETGAYCMIDMHNFARY NGGIIGQGGVSDDIFVDLWVQIAKYYEDNDKIIFGLMNEPHD LDIEIWAQTCQKVVTAIRKAGATSQMILLPGTNFASVETYVST GSAEALGKITNPDGSTDLLYFDVHKYLDINNSGSHAECTTDN VDAFNDFADWLRQNKRQAIISETGASMEPSCMTAFCAQNK AISENSDVYIGFVGWGAGSFDTSYILTLTPLGKPGNYTDNKL MNECILDQFTLDEKYRPTPTSISTAAEETATATATSDGDAPS TTKPIFREETASPTPNAVTKPSPDTSDSSDDDKDSAASMSA QGLTGTVLFTVAALGYMLVAF |
| SEQ ID NO: 9 - *Trichoderma reesei* | 5 | MMLSKSLLSAATAASALAAVLQPVPRASSFVTISGTQFNIDG KVGYFAGTNCYWCSFLTNHADVDSTFSHISSSGLKVVRVW GFNDVNTQPSPGQIWFQKLSATGSTINTGADGLQTLDYVVQ SAEQHNLKLIIPFVNNWSDYGGINAYVNAFGGNATTWYTNT AAQTQYRKYVQAVVSRYANSTAIFAWELGNEPRCNGCSTD VIVQWATSVSQYVKSLDSNHLVTLGDEGLGLSTGDGAYPYT YGEGTDFAKNVQIKSLDFGTFHLYPDSWGTNYTWGNGWIQ THAAACLAAGKPCVFEEYGAQQNPCTNEAPWQTTSLTTRG MGGDMFWQWGDTFANGAQSNSDPYTVWYNSSNWQCLVK NHVDAINGGTTTPPPVSSTTTTSSRTSSTPPPPGGSCSPLY GQCGGSGYTDRKIKCDRALPACTNCRQRNVPCPGYSIRLS WPRPGDKRRAVELKSTAKQKKHMLASLYQHSLFLNVTS |
| | | WDVNTFYQLSEARSDMEIPKISIPPALPSIPSMRVHESQSGL LQYFMDMGPQLLVMDSSGDLTDWILRLAFYDATPASNAVLH SLLALSSLNLGRNSVAMMYKIMALSYLQQSLQLGNIGHRTA QHLMTSMLLYLCETFHISNTTKDWAEYLCGAKQIVSTGVLAR EVHIDVSLLFDWVSYHETFARFGLVYWKPAVALRGLRFCAM SPETAQVFAPRNDFVCDRDVLGYTSSIFSSLTPRIKDNSHLS HLETQELYALEAKLRGGLMDFNPLNTLPDEAPKERDRITELY RLAALIYLNRAALGYSGEELRHRNLVNRGLDILCGKLTSTPP WPVFMIACEARDDAQRMTALEILDRAGEEPRSFNMRLIRQL VEACWNQDDLHEDEPLDYHTKLRIAIRAAPYVPPFS |
| SEQ ID NO: 10 - *Aspergillus niger* | 11 | MVSFLGQARLAVPILSAFAWMFAASSAIPPPPPGTLSPERLQ WIREVIGNQTEGGSISDIAKRSTILHTSQDGVDSGGFYYSVY NDNGASVGYTEYPTTGQFELGWNTETEFLGGKGFKGGSTR SLTWDGYFTAEGDWTLAIYGWTLNPVTEWYIVESHGTGTP GNGNILGQVDSDGGVYDVYNLPYRNVPEIYGVTNFDQHWS VRRSHRSTGTVDVSTHFQRWKELGLNPGTPIFQMVTLEGFS GQGYLDFTVQA |
| SEQ ID NO: 11 - *Aspergillus niger* | 11 | MLTKNLLLCFAAAKAVLAVPHDSVVERSDALHKLSERSTPSS TGENNGYYYSFWTDGGGDVTYTNGNAGSYSVEWSNVGNF VGGKGWNPGSAKDITYSGTFTPSGNGYLSVYGWTTDPLIEY YIVESYGDYNPGSGGTYKGTVTSDGSVYDIYTASRTNAPSIQ GTATFTQYWSVRQNKRVGGTVTTSNHFNAWAKLGMNLGT HNYQILATEGYQSSGSSSITIQ |
| SEQ ID NO: 12 - *Aspergillus niger* | 11 | MVAYSSLLLALSAASSAVALPNGKALLDMAKRSLTSSAEGE SNGYFYSFWTNGEGDVTYTNGDGGEYTVEWTDCGNFVAG KGWNPGSAQAVTYSGSWETDANAYLSVYGWTTSPLVEFYI VDKYGDYDPSSGLTELGTVDSDDGTYKIYQTTREDADSIEG TATFKQYWSVRTEGRIGGTVTTQNHFDAWENLGLELGTFN YMIVATEGYESSGSATITVDASSDSTEESC |
| SEQ ID NO: 13 - *Aspergillus niger* | 11 | MKVTAAFAGLLVTAFAAPVPEPVLVSRSAGINYVQNYNGNL GDFTYDESAGTFSMYWEDGVSSDFVVGLGWTTGSSNAITY SAEYSASGSSSYLAVYGWVNYPQAEYYIVEDYGDYNPCSS ATSLGTVYSDGSTYQVCTDTRTNEPSITGTSTFTQYFSVRES TRTSGTVTVANHFNFWAQHGFGNSDFNYQVMAVEAWSGA GSASVTISS |
| SEQ ID NO: 14 - *Aspergillus niger* | 10 | MVQIKAAALAMLFASHVLSEPIEPRQASVSIDTKFKAHGKKY LGNIGDQYTLTKNSKTPAIIKADFGALTPENSMKWDATEPSR GQFSFSGSDYLVNFAQSNNKLIRGHTLVWHSQLPSWVQAIT DKNTLIEVMKNHITTVMQHYKGKIYAWDVVNEIFNEDGSLRD |
| | | SVFYKVIGEDYVRIAFETARAADPNAKLYINDYNLDSASYPKL AGMVSHVKKWIEAGIPIDGIGSQTHLSAGGALNALAGAGT KEIAVTELDIAGASSTDYVEVVEACLDQPKCIGITVWGVADP DSWRSSSTPLLFDSNYNPKPAYTAIANAL |
| SEQ ID NO: 15 - *Aspergillus niger* | 5 | MRISNLIVAASAATMVSALPSRQMKKRDSGFKWVGTSESGA EFGSALPGTLGTDYTWPETSKIQVLRNKGMNIFRIPFLMERL TPDGLTGSFASTYLSDLKSTVEFVTNSGAYAVLDPHNYGRF DGSIIESTSDFKTWWKNVATEFADNDKVIFDTNNEYHDMEQ SLVLNLNQAAINGIRAAGATTQYIFVEGNAYTGAWDWT TYNDDLSGLTDSEDKIIYEMHQYLDSDSSGTSETCVSSTIGK ERIEKATEWLKTNNKQGIIGEFAGGVNSVCEEAVEGMLAYM SENSDVWVGASWWSAGPWWGTYMYSLEPTDGTAYSTYLP ILEKYFPSGDASSSSSASASVAAATSAVSTTTTAAFEQTTTP ATQVEIASSSSSSSAVAASQTTLSKVKSKSKSPCKLSSATSS AVSSAAAVTTPAVAATTPAAAPTSSSVAFATTSVYVPTTTAA APSQVSSSAAASSSGVVGVSDPQGPSATNSAGEVNQYYQC GGINWTGPTVCASPYTCKVQNDYYYQCVAE |
| SEQ ID NO: 16 - *Aspergillus niger* | 5 | MKTLSLVAVLLVQAWTASSSPRAKRSSGFQWFGSNESGAE FGSGNIPGTLGTDYIWPDATAIETLHSAGMNIFRVPFLMERL VPNELTGAADATYMADLKATVQTITDLGAYAVVDPHNFGRY YGNIITSTSDFAAFWTTVASEFTDNDLVIFDTNNEFHDEDQTL VLNLNQAAITAIRATGATTQYIFVEGNSYTGAWTWTTTNTNL VNLTDPNDHLIYEMHQYLDSDGSGTSSTCVSSTIGAERIADA TSWLRENGKQAVLGEFAGGANSVCEEAVTGMLDAMVDAS DVWLGGMWWSAGPWWGDYIFSLEPDDGVAYEYYLSVLEG YTPGSSSSSGGTTTTAAASTSSTSAAGSATTSKVTTTTAAAT ATSTAVAAHYAQCGGQGWTGATTCADGYTCTVENAWYSQ CL |
| SEQ ID NO: 17 - *Aspergillus niger* | 7 | MHQRALLFSALLTAVRAQQAGTLTEEVHPSLTWQKCTSEGS CTEQSGSVVIDSNWRWTHSVDGSTNCYTGNTWDSTLCPD DETCATNCALDGADYESTYGITTDGDSLTLKFVTGSNVGSR VYLMDTSDEKYQTFDLLDAEFTFDVDVSNLPCGLNG ALYFTAMDADGGTSRYPANKAGAKYGTGYCDSQCPRDLKF INGEANVDGWEPSSNNDNTGIGNHGSCCPEMDIWEANSIST ALTPHPCDSSKQTMCEGNNCGGTYSDDRYGGTCDPDGCD FNPFRMGNETFYGDGKIVDTGSKMTVVTQFITDGSSSLSEIK RYYVQNGNVIANAESSISGVTGNSITTDFCTAQKKAFGDDDV FAAHNGLAGISDAMSSMVLILSLWDDYYASMEWLDSDYPEN ATATDPGVARGTCDSESGVPATVEGAHPDASVTFSNIKFGP INSTFSASA |
| SEQ ID NO: 18 - *Aspergillus niger* | 7 | MSSFQIYRAALLLSILATANSQQVGTYTTETHPSLTWQTCTS SGSCTTNNGAVVIDANWRWVHSTSSSTNCYTGNEWDTSIC TDDVTCAANCALDGATYEATYGVTTSGDELRLNFVTQGSSK NIGSRLYLMSDDSTYEMFKLLGQEFTFDVDVSNLPCG LNGALYFVAMDADGGTSKYSGNKAGAKYGTGYCDSQCPR DLKFINGEANCDGWEPSSNNANTGVGDHGSCCPEMDVWE ANSISNAFTAHPCDSTSQTMCDGDTCGGTYSASGDRYSGT CDPDGCDYNPYRLGDTDFYGPGLTVDTNSPFTVVTQF ITDDGTSSGTLTEIKRLYVQNGKVIANGASTYSSVNGSSITSD FCEAQKTLFGDENVFDTHGGLAGMGEAMANGMVLVLSLW DDYASNMLWLDSDYPVDSSASTPGVARGTCSTDSGVPATV EAESPNAYVTYSNIKFGPIGSTYSSGSSSGSGSSSSSSSTT TKATSTTLKTTTTTSSGSSSTSAAQAYGQCGGQGWTGPTT CVSGYTCTYQNAYYSQCL |
| SEQ ID NO: 19 - *Aspergillus niger* | 16 | MPTSSLLWSVGSLALSSMILPAAASGYQLVETWKGEDFLTA FDFYTGADPTNGFVTYANQSYAESKGLVKVNSNGSFYMGV DHTTKLSTNGPGRESVRIGSNKYYDEGLFIIDLEHMPGSVCG TWPAFWSTGKDWPTDGEIDIIEGVNKNEANEIVLHTSGTC QVSSQKMTGTLSSTECGEDSGTTGCVVEGTQGSSGNPFNE NGGGVYAMQWTDEFLKFWFFPRGSIPTSITKGDPDVAAFGT PMAHMQGSCSIAEHFKAQQFIFDTTFCGDWAGGVYSTSGC PVSDSSSSFKSCVAYVAENPAAFAESYWEINYIKIYQTGVAA SASASASRVESAATVVETVSSVKEVTNSVVATTAAATTVATT ADAETATTMTIAETTTAAAATESSVADENGSSASTSTHYVTM TTTICPIAESSSAAADLAGGSSEATEGSNSEGSSATATPDSV TGASAESNGSEGSSATESTPSTATGASAEANGNESSSTSEA SSVAGTPSDISTTGASAEANGSEDSSASSEAKAVASATPSS VTGASAEANGNDSASSNAATTSNVSGASTQAGDDESTPAS AVANAGSSATPASVSGASAEANGSEQSAAAAASSVSGASA VANGSEGSSSQSSGSQAGSDSYGSVPSSAAAYGRPAPSSS SHAFSTAPSSSGSIRQPTSAAAANNDAAATQGSSASGSKSG HFGSGSSSATTPSTPVFTGGANKLTLGASSVVGVLAFALLA |
| SEQ ID NO: 20 - *Aspergillus niger* | 16 | MRRTATLLSALGLTAQLSSAAYTLQDEYSGDSFFDGFTFFT GADPTNGFVDYVDEATAQSNGYISTSGDAPVYMGVDHTNIA GSSGRQSVRISSDATYNHGLFILDLGHMPGGVCGTWPAFW LVGADWPNNGEIDIIEGVNQQSGNDMTLHTSDGCSVSSSAD FTGSMTTDNCYVSAAGQSNNAGCGITDPDGTSYGTAFNAN NGGVFATEWTSDAISIWFFERGSIPDDIDSGNPDPDSWGSP VARFQGDCDIDAHFDNLQIIFDTTFCGDWAGNV WGSGSCASVASSCSDYVANNPEAFAEAYWIINSLKVYQDD GVDDDTVAGVVDYGDDEDDEDDQDDDNDDDDSDDDDDSD |
| | | DDDDDDSDSDDDSDDDDDDDDDDDDDDDDSRPWRGGG RNHHGSKLNASTSTSSFPSPSFVRKARWERRNPMAGMIF |
| SEQ ID NO: 21 - *Aspergillus niger* | 17 | MLSKMQLAQLAAFAMTLATSEAAYQGFNYGNKFSDESSKF QADFEAEFKAAKNLQTTSGFTSARLYTMIQAYSTSDVIEAIPA AIAQDTSLLLGLWASGGGMDNEITALKTAISQYGEELGKLVV GISVGSEDLYRNSVEGVEADAGVGVNPDELVSYIKEVRSVIS GTALADVSIGHVDTWDSWTNSSNSAVVEAVDWLGFDGYPF FQSSMANSIDNAKSLFEESVEKTKAVAGDKEVWITETGWPV SGDSQGDAVASIANAKKFWDEVGCPLFGNVNTWWYILQDA SPTTPNPSFGIVGSELSTTPLFDLSCKNSTTSSSSSVVSAAA SSAAGSKAVGSSSGAQASATPSFTVGRPGVNGTVFGNGTY PLRPSGSASARPSAGAISSGSGSGSSGSGSSGSTGTSATS GQSSTSGSAAAGSSSPAAFSGASTLSGSLFGAVVAVFMTLA AL |
| SEQ ID NO: 22 - *Aspergillus niger* | 17 | MAGVNRSFSYSRGDDALLRDDEREISPLRSAEDGLYSTSYG DISPLSAGFQGQTRPFDRGLVSVPEGQSLERHMTSTPGMD NLGPASVGGGISGIALGVANSNNRQSGVDAFR ETDVPVRNLPAERDFNTTGSDNPYIPAPPDGDIYPNSETMR YRDSYSSHTGLGAGAPFAEHSTPGTTPSQRSFFDSPYQGV DAGPYQRHSAYSSHDYPLVINPDDIADDGDDGF PVHPKGVADYRSNGNVPAAGVAGAAAAGGLLGKFRALFKK EQPSPYYDSDIGGGLGGAEKAQGGRHIIGGGSRKRGWIVGL VLAAVIVAAIVGGAVGGILGHQEHDGDTSSSS SSSGTGSGGSDKGDGLLDKDSDEIKALMNNKNLHKVFPGV DYTPWGVQYPLCLQYPPSQNNVTRDLAVLTQLTNTIRLYGT DCNQTEMVLEAIDRLQLTNMKLWLGVWIDTNTTTTDRQISQ LYKIVESANDTSIFKGAIVGNEALYRAGSDVASAETNLIGYIN DVKDHFKEKNIDLPVGTSDLGDNWNAQLVSAADFVMSNIHP FFGGVEIDDAASWTWTFWQTHDTPLTAGTNKQQIISEVGWP TGGGNDCGSDNKCQNDKQGAVAGVDELNQFLSEWV CQALDNGTEYFWFEAFDEPWKVQYNTPGQEWEDKWGLM DSARNLKPGVKIPDCGGKTIT |
| SEQ ID NO: 23 - *Aspergillus niger* | 5 | MKFSSSLLTLASLALANLSTALPKASPAPSSSSSSSSSASTS FASTSGLQFTIDGETGYFAGTNSYWIGFLTDDSDVDLVMSH LKSSGLKILRVWGFNDVTTQPSSGTVWYQLHQD GKSTINTGADGLQRLDYVVSSAEQHDIKLIINFVNYWTDYGG MSAYVSAYGGSDETDFYTSDTMQSAYQTYIKTVVERYSNSS AVFAWELANEPRCPSCDTSVLYDWIEKTSKFIK GLDADHMVCIGDEGFGLNTDSDGSYPYQFAEGLNFTKNLGI DTIDFGTLHLYPDSWGTSDDWGNGWISAHGAACKAAGKPC |
| | | LLEEYGVTSNHCSVESPWQKTALNTTGVSADLFWQYGDDL STGKSPDDGNTIYYGTSDYECLVTDHVAAIGSA |
| SEQ ID NO: 24 - *Aspergillus niger* | 5 | MRGKPPFVDNVPRRMHPSVEHIDTCVPVTALKALELRDVKL LLQAQGTYIRLIDEARGNLLAELKAFKRNHVHGFVALSQQEQ AAGPSHARFIAWGGQSVRAFDLFLSVQEPSARLVASSAEYQ GPDWILAGCASSQEESTTAYVLTAHNAVLGLHIVDGDG SKHEKAVHIKPLVTGVNTILYSADIVALSDSHILIAAGTVFGEII VWSSYLNESGGAVGSIHHFFTGHEGSIFGVRISPSIASLRSD QSGRLLASCSDDRTIRIWDISDCERTSRHDAPAYSTDGFELR STGFGAVAHDNEVGSEKTIASAFGHLARIWGVNFLPVGEGL GKVNLVSRGEDATCILWDLTWDPSSQTPQFKLTEGISIRKHT GKHVWSLEVRSTDTEAVIYTGGADGAVKNFTIKADENGSLV LPNLHDRITMTVDTQNPEPHVETSIKGFDFVSQNKFIAVTAR GEVQVGHINAQDLSKQHIFKETLFVEDDLRSYSVVG SLPQEGIALVGNARGSIRLYNHNVKTLIRLVDVGQRPLILIPLD YNEVSESEANLVFTVSYPNSEIAELFKVSLSGDGPCVDRITIN LPYGFGMASASLVCGKQYLALGSLTGNFALYKVANTESIEPV MPPSKLHGREGLSYITSFSSLYGEEEQLHPYFLTCGRSGTY GVHKLVISEGAEPSISLQTLHLASLAFNCEIQGAYIDKVSQDL MIYGFQPMDFVLWNESAQTEVARHKCNGGRRAWAFQPNN TKAWAGSLLWIQSGFSGMEIHPDASRTLRSGSHGREVKAM SATQQGGLGPLFATGSEDTNVRIFTPRDPQSENRWGAFK CLRLLREHHTGLQSVGWSTDGRFLFTSGGYEQFFVWRTHS VPRFGLGTTSDGECPKSDPNSDLRTTSFDVLEVEDEQAEGS FLICLTYSNSTIKIFHYTSSTEGGSFTLLANGTYMSNCLTQAN FLRNGSSLSLITASTDGYFTLWDLTSVLEPFYTITSPLRMKQT IQGASITPATISCENRYQIHWNSIKSLELADLSNTLSLIVAAGD DNSMTVTLLDTSIENGAATCTIAVPDAHAACITTTKILTQHVN PSDGTARFAVASSGNDHQVKIWWVDIDTKMSGADRIQISKA AERYSSVADISSLDVLHDASGRIGTTTNGPNWIGYLTTTENA SLVLNYNFAAGGATIDNALVPAYPGDLASQFRLFEDVYAQK PETAPWDAKDAVFGVWIGINDIGNAYYSTDAATYTPKLISRL ESLVEEVYKNGGRKFLFLNVPPTSRSPLFLDQGEEVV KQHAEYLAVFNENLEGMVEKFNKEKGDVTTVLYDSWSFMT KILDDPTAYGFPNATCIDDDGTSCGSLTERSPCFDFIRPIGID DICRCEDLQHFLFEYPLFSTFGRFNLNKRRR RCLAKLVTSVLDWDSWVIMKFQSTLLLAAAAGSALAVPHGP GHKKRASVFEWFGSNESGAEFGTNIPGVWGTDYIFPDPSAI STLIDKGMNFFRVQFMMERLLPDSMTGSYDEEYLANLTTVIK AVTDGGAHALVDPHNYGRYNGEIISSTSDFQTFWENLAGQY KDNDLVMFDTNNEYHDMDQDLVLNLNQAAINGIRAAGATSQ |
| | | YIFVEGNSWTGAWTWVDVNDNMKNLTDPEDKIVYEMHQYL DSDGSGTSETCVSETIGKERVTEATQWLKDNKKVGFIGEYA GGSNDVCRSAVSGMLEYMANNTDVWKGASWWAAGPW WGDYIFSMEPPDGTAYTGMLDILEAYL |
| SEQ ID NO: 25 - *Rasamsonia composticola* | 5 | MKFSTVVCGLAAAGGALAAPAKERSIKKRASPFQWFGSNES GAEFGQNNIPGVEGTDYTFPNTTAIQVLIDQGMNIFRVPFLM ERMVPNQMTGPVDSAYLQGYSQVINYITSHGASAVIDPHNY GRYYNNIISSPSDFQTFWNTIASNFADNDGVIFDTNNEYHDM DESLVVQLNQAAIDGIRAAGATSQYIFVEGNSWTGAWTWTQ VNDAMANLTDPQNKIVYEMHQYLDSDGSGTSDQCVNATIG QDRVASATAWLKQNGKKAILGEFAGGANSVCESAVTGLLD HLADNTDVWTGAIWWAAGPWWADYIFSMEPPSGIAYEQVL PLLQPYL |
| SEQ ID NO: 26 - *Rasamsonia composticola* | 7 | MDRILALVLLPLAAVTAQQIGTIPEVHPKLTTWKCTTAGGCV QQDTSVVLEYLSHPIHEVGNSDVSCVVSGGLNQSLCPNEEE CSKNCVVEGANYTSSGVHTDGDALTLNQYVQNGDQVVAAS PRVYLLASDDEDGNYSMLQLLNQELSFDVDVSKLVCGM NGALYLSEMDVSGGRNSLNPAGAQYGSGYCDAQCGVQPFI NGTVNTGSLGACCNEMDIWEANALATAYTPHPCSVASIYPC SGDECGSNGVCDKPGCGYNPYALGDHDYYGYGKTVDTSK PFTVVTQFLTDDNTTTGTLTEIRRLYVQDGTVIQPSPSDS VSSLTDSFCSTADSYFEPLGGMKGMGEALGRGMVLVFSIW NDPGQFMNWLDSGNAGPCNSTEGNPATIQAEHPDTAVTFS NIRWGDIGSTFQS |
| SEQ ID NO: 27 - *Rasamsonia composticola* | 10 | MVRLSPVLLASIAGSGLPLYAQAAGLNTAAKAVGLKYFGTAT DNPELSDTAYETELNNTQDFGQLTPANSMKWDATEPQQNT FTFSGGDQIANLAKANGQMLRCHNLVWYNQLPSWVTGGS WTNETLLAAMKNHITNVVTHYKGQCYAWDVVNEALNDDGT YRSNVFYQYIGEAYIPIAFATAAAADPDAKLYYNDYNIEYPGV KATAAQNIVKLVQSYGARIDGVGLQSHFIVGQTPSTSAQQQ NMAAFTALGVEVAITELDIRMQLPETSAQLTQQATDYQSTVQ ACVNTDSCVGITLWDWTDKYSWVPSTFSGWGDACPWDDN YQKKPAYNGILTALGGTPSSSTSYTLTPTTTSSGGSGSPTDV AQHWEQCGGLGWTGPTVCASGFTCTVINEYYSQCL |
| SEQ ID NO: 28 - *Rasamsonia composticola* | 12 | MLVSPSFKMKPFIAVALSLPLAATLTTAAPTPTKTLQRRADF CGQWDSTVTGSYTVYNNLWGEGSASSGSQCTGVDSLDGN TLAWHTLWTWEGGSSSVKSFANAAYAFTATQLSQISSIPTT WQWSYTGTSIDADVAYDLFTSSSPSGSNEYEIMIWLAALGG SFPISSTGTTPIASPTIGGVSWNLYSGPNGATTVYSFVAA |
| | | TEQTDFSGDINDFLAYLQENQGLSSSQYLLSIQAGTEPFTGE DAELKTTAYSVSVLTS |
| SEQ ID NO: 29 - *Rasamsonia composticola* | 5 | MKTVTTLSTLCALCGLATAWLPGIHKDITSIDGRNHFNTSSD NKRWLPASGKIRGVDLGSLFVTEPWMASNAWSSMGCGNA PSEFDCVMALGQDAANSAFKNHWSSWITKDDITEMQSYGL NTIRIPVGYWIKEDIVYSDSEHFPQGGLSYLEQVCGWASDA GFYIIIDLHGAPGAQVPKNADTGQYAPTAGFYVDYQY ERALEFLEWMTTNIHTNNNFRNVGMLEIVNEPIQDANQVAS MRTSYYPNAFKRIRAAEQKLGITQNNYIHIQMMNAKWGSGD PNEALTDTYFAAYDDHRYLKWDTSVPVSKDSYLSTSCNDDR GGNTPTIVGEFSLSPPDNVQWTSDWDPNSNKDFYQKWFAA QVMSYEKQNGWIFWSWKTDLGDYRWSYKQAVEAGVIPKG LDSVYNMGAC |
| SEQ ID NO: 30 - *Rasamsonia composticola* | 5 | MQVSRIAALAALLQGAWAQAGPYAQCGGIGYSGSTVCAAG YTCTSQNPYYYQCVPATATTTTTTSSASASSTSSAPSTTCA GTFTPISAADFVANLNPGWNLGNTLDATPDEGSWNNPPVV SSTFDEVKAAGFKSVRLPVTYAYHFIGDSPDWTI NATWLQRVSDVVDMITSRGLYTIVNAHHDSWIWADVTQPGA NLTMIEEKFYRLWYQVGSKLACKSSLVAFEPINEPPCNDATD AAEINKLNTIFLKAINDAGGFNSQRVVTLVGGGEDSVKTSEW FVAPTGYSNPYAIQFHYYNPYDFIFSAWGKTIWGSDSDKSTL STDLQLIRNNFTTVPLLIGEYDASPTNCETAARWKYFDYLIRT ARALNISTIMWDNGQDHLDRTTGVWRDPSAVDIIMDATSGIT NSLPDSTEDPSATTQWSSAYIFHKYGDPVSDQSLPFLFNGN SVSSISASDGTKLTPETDYVVAGSNITFKASFLSKYLSSTTAP GILANLTVSFSAGASEVIQLVQWTTPSLSSTSVAASA VNGSDLYIPITWGGIPKPAAVKAVEANGEHLVDSWTEYLPVI QQGRTTYSSQWNWDDSHIIITAATISTVLAAGQTTVFTFEFY PRDNGVVNAVNFTLTV |
| SEQ ID NO: 31 - *Rasamsonia composticola* | 45 | MFTSQEDRESIKSDEKEAVPPIDLSAVAIVFVMNLKLFVSVSA LLLTPLASALTGTTTTTWDCCKPACSWTQNAQAGGASGTV ATCNINNQVLSDGATAQSACQGGNAYSCSDFQPIIISDTLSY GFAGNWETSNCCKCFQFTWTSGEGAGKSMIVQVV NSGGVNTGDFDIYTPGGGVGDYNACTSQYGAPPQGWGNQ YGGVSSDAECDQLPSNLQPGCHWRFEWAGGGINGWTTNY EEVECPSQLTSISGCYP |
| SEQ ID NO: 32 - *Rasamsonia composticola* | 5 | MRYRDGEAVAFVRHDADVQQALDLTEPGLAPDAAVRPPPP RVLQSWSLIMNEQSGTFLFLASGFCRRHLLDGETMDRRLDF VRFFAPFITPRRGNALPAAFLWRTHSDRPDSDEGVSTILIRH PSILALIHDYSSMHVAHRWAVMSVDVLLPRRAGYLA |
| | | WYTSLLPGLDLGYRPLQPASSSEDNLPPRTMAVTIRRFTRR SLLVLVSKTTILVTLVSLLLWLLVNTNIESAFDLLNLLPLPSPS PSPALAYTDGGYSLQRHSSQPPEAGSR SSSSASHNYTPPFHTAGRYILDAQNQRVKLASINWYGGSDE DFVPSGLDVQPRDRIAALIRDLGFNSVRLPYSDEMVRDNPLI PASRLAANRDLVDPDTGGASARDVFAAVVESLTDAGLLVIV NNHITQATWCCGANLCDAGWANDWFGGQWFCRVS QTTEEWIEHWETVMRPLAHNPRVIGADLRNEPRGLWGTLH WDDWVAAAERAAERLLALNPDWLIIVEGISSANDLSGVRTR PVRLPPPFAADRVVYSAHVYSWSGWGSLYPYSRRTYEDFV ASMRGNWAYLLEEDLAPVWVGELGTPDQPTEGDCNYWT HLVEFLRVTDASWGYWALNPRKPADHEWESYGLTCNDWA CVPVSHPVINWTRRRDEVHGATMTNSDSGPLAETSGSMLA PVACLEAAPPLPATAFSPSRPRSARPSVCLSVCLSVCLSVCL SVCRARARPRTDTAKSITHQKLRWKNPMLCSTDDGGYPAA WRWASHWRTSLSRTPRLARARTHDRSSGGAPHLANARGV CYRQFGEGFLPMTPRARAPSPPFSVPIGQSVLAADTDDITQ GLKTPWFYGQRRREYTGSGGALRKVETGNGYNNRHPAHA RGLQCEWPLGIAPALRPPLGRGHVGSCFPAQSPARRPPRP PNSARPSPQPPPSNEIAMMCSRRRLTGEESGRSPAALCPP GRVWVQTPRVAPSQPLIIAPCLSSAHVVHAQKLALAGSSAD AAAGRPWLREDSAAIEPGAEDLHRVHAHRPRQCPDLRHAP VLGAVPPLEGDPGRVATTAVGQEHCQRGWERDTAVRRQS AGDADIALLAASDDVRRPGPTGGGGGWRGGACDHPAELLR GSRHAADHVERARQSSPGGRPVYLAMEQKETKAPEKAATLI SMFQGQFRDICSTFHPAGLPGEIAGKSSNVAFAARHIIEAHR RDETVNPRDVIITVMDADTHLSQDYFTEIRRLHYRHIDEADR SFYASPIIFDRNSNEVPVFPHHDPHVGLFAAPGPRGEGWWV GWGSHGDWRGHAHAAQMLLLHGRGHHFAGDLHPGQPV RLEQRRRGLAAFAGDLHGAVSPGPAAHVGSAGHGVCGAA EPGPVDRQGAAAHAPAAAAPGVVPSAVGGAFPAVPFHDPA AVLVDLHVCDAGVADPPDAGVDVLVHGGAAVRVVRVDEHL PDAVRSVARGVCQRARAGHGAGGTAGCGLLVPDVVAWEA PHRAGVLPDRGDDLRVDSVGACGDFAFLDGAAGVSG |
| SEQ ID NO: 33 - *Rasamsonia composticola* | 5 | MAAGFLRVSGTKIVDANGTPVLLRGTALGGWMNMENFITGF PGHESTHRAAMLAVLGKEKYDYFFDKFLEYFFTEKDAQFCK SLGINCLRLPFNYRHFEDDMNPRVLKEEGFKHLDRVVDLCA KYQIYTILDMHTLPGGQSPGWHADNLTSHASFWDHRDHQD RTVWLWQQIAARYKSNPWIAGYNLINEPCDPQHVRLPAFYV RLEKAIHEIDPDHILFLDGNTFAAEWKGFDEYPILPNAVYSLH DYSRLGFPGPEQFTGSQEQLERLERQFLRKAEFHLSRNAPI |
| | | WNGEFGPVYANPQYEEDADAINRQRYELLNAQLRLYDKYAI PWSIWLYKDIGLQGMLHTSPSSPWNKLLAPFLEKKKTLQLD AWGKYPSQEIDALLGPLVEWIDRVSPTAKSTYPSTWDTRRH IERAVLQTFLAESFQKEFAELFRDKSMKELDELARSFAFE QCVQRTELNKILSKHAEASGLA |
| SEQ ID NO: 34 - *Aspergillus niger* | 11 | MLTKNLLLCFAAAKAALAVPHDSVAQRSDALHMLSERSTPS STGENNGFYYSFWTDGGGDVTYTNGDAGAYTVEWSNVGN FVGGKGWNPGSAQDITYSGTFTPSGNGYLSVYGWTTDPLIE YYIVESYGDYNPGSGGTYKGTVTSDGSVYDIYTATRTNAASI QGTATFTQYWSVRQNKRVGGTVTTSNHFNAWAKLGMNLG THNYQIVATEGYQSSGSSSITVQ |
| SEQ ID NO: 35 - *Aspergillus niger* | 11 | MVAYSSLLLALSAASSAVALPNGKALLDMAKRSLTSSAEGE SNGYFYSFWTNGEDVTYTNGDAGEYTVEWTDCGDFVAGK GWNPGSAQAVTYSGSWETDANAYLSVYGWTTSPLVEFYIV DKYGDYDPSSGLTELGTVDSDDGTYKIYQTTREDADSIEGT ATFKQYWSVRTEGRIGGTVTTQNHFDAWENLGLELGTFNY MIVATEGYESSGSATITVETSSDSTEESC |
| SEQ ID NO: 36 - *Aspergillus niger* | 11 | MVSFLGQARLAVPILSAFACMLAASSAIPPPPRGALSPERLQ WIREVIGNQTENDSVSDLAKRSTVLSTSQDGVDSAGFYYSV YNDNGADVGYTEYPTTGQFELGWSAEAEFLAGKGFKGGNP RSLTWDGYFTAEGDWTLAIYGWTTNPVTEWYIVETHGTG TPGNGDILGQVDSDGGVYDVYNLPYRNVPEIYGVTNFDQY WSVRRTHRTTGTVDVSAHFQGWKNLGLNPGSPVFQMVTL EGFSGQGYLDFTVSA |
| SEQ ID NO: 37 - *Aspergillus niger* | 10 | MKTFGAASLITALLAASEGANAFTKTNTSPLKEVAESKGILIG SGAVNPNYLNDSLFATVLADQFQSLAPENELKWTFIHPTPN QYNWNATDRLVSFAEKHSMVVKGHGLISSCCNPDYLLNITD PKQFRAAMVTHFDAIMHRYAGKMDRWDVVIEALKTMGGGL QVNDFYKVLGPGYIADAFHIARAAGPEAKLFLSESLVETYPG KRQELYDLVSGLVSDNVPIDGVALQMHVTEAAPIPGVITDIVK SYNALGLEVSISEMDVHTLNTTLETDIYNAVLTEALGAGITDIT FWGFTDKHAYTWIKGAKPLMFDEYYNPKGEFYATHTALTSF DNTTSPK |
| SEQ ID NO: 38 - *Aspergillus niger* | 10 | MVQIKVAALAMLFASQVLSEPIEPRQASVSIDTKFKAHGKKY LGNIGDQYTLTKNSKTPAIIKADFGALTPENSMKWDATEPSR GQFSFSGSDYLVNFAQSNNKLIRGHTLVWHSQLPSWVQSIT DKNTLIEVMKNHITTVMQHYKGKIYAWDVVNEIFNEDGSLRD SVFYKVIGEDYVRIAFETARAADPNAKLYINDYNLDSASYPKL TGMVSHVKKWIAAGIPIDGIALNALAGAGTKEIAVTELDIAGA |
| | | SSTDYVEVVEACLNQPKCIGITVWGVADPDSWRSSSTPLLF DSNYNPKPAYTAIANAL |
| SEQ ID NO: 39 - *Aspergillus niger* | 17 | MAGVNRSFSYSRGDDALLRDDEREISPLRSAEDGLYSTSYG DVSPLSAGVQAQNRPFDRGLVSVPEGQTLERHMTSTPGMD NLGPASVGGGISGIALGVANSHNRQSGVDAFRETDVPVRNL PAERDFNTTGSDNPYIPAPPDGDIYPSSEAVRYRDSYSSHT GLGAGAPFAEHSTPGTTPSQRSFFDSPYQGVDAGPYQRHS AYSSHDYPLVINPDDIADDGDDGFPVHPKGAADYRSNANVP GTGVAGAAAAGGFLGKFRALFKREEPSPFYDSDIGGGLGG AEKAQGGRHIIGGGSRKRGWIVGLILAAVIVAAIVGGAVGGIL GHQEHDGDTSSSSSSSSSSSGTGSGGSDKGDGLLDKDSD EIKALMNNKNLHKVFPGVDYTPWGVQYPLCLQYPPSQNNV TRDLAVLTQLTNTIRLYGTDCNQTEMVLEAIDRLQLTNMKLW LGVWIDTNTTTTDRQISQLYKIVENANDTSIFKGAIVGNEALY RAGSDVASAETNLIGYINDVKDHFKDKNIDLPVGTSDLGDN WNAQLVSAADFVMSNIHPFFGGVEIDDAASWTWTFWQ THDTPLTAGTNKQQIISEVGWPTGGGNDCGSDNKCQNDKQ GAVAGIDELNQFLSEWVCQALDNGTEYFWFEAFDEPWKVQ YNTPGQEWEDKWGLMDSARNLKPGVKIPDCGGKTIT |
| SEQ ID NO: 40 - *Aspergillus niger* | 16 | MPTSTLLWSVGSLALSSMVLPAAASSYQLVETWKGEDFLTA FDFYTGADPTNGFVTYANQSYAESTGLVKVNSNGTFYMGV DHTTKLSTNGPGRESVRIGSNKYYDEGLFIIDLQHMPGSVC GTWPAFWSTGKNWPADGEIDIIEGVNKNEANEIV LHTSGTCQVSSQKMSGTLTSTECGEDAGTTGCVVEGTQGS SGTPFNENGGGVYAMQWTDEFLKFWFFPRGSIPSSITKGD PDVAAFGTPMAHMEGSCSIAEHFKAQQFIFDTTFCGDWAG GVYSTSGCPVSDSSSSFKSCVAYVAENPTAFAESY WEINYIKIYQTGVAASASASASHVESAASVAETISAVREGTN SVVATTAAATIAVTNTADAETATTMAEATTVAASTETSAADE NGSSASTSTHYVTMTTTICPIAESSSAAAALAGGSSEATEAS NSEGSPAAATPSSVTGASAEANESESTSAAVTTPS TATGASAEANGSESSSASEAGSVAGATPSSVSTTGASGEA DSSEGASAAATPSNVSSTGASAEANGSEDSSASSEAKTVAS ATPSSVTGASAEANGNDSASSNAATASNVSGASAQAGDNE STPASAGANAGSSAAPSSVSGASAEANGSEGSSSHSS GSQAGAHSYGSVPSSAAAYGRPAPSSSSHAFATAPSSSGS SRVPTSAAAANNAAAATQGSSASGSNSGSSGHGSSSATTP STPVFTGGASKLTLGASSVLSVLAFALLA |
| SEQ ID NO: 41 - *Aspergillus niger* | 16 | MRRTATLLSALGLTAQLSSAAYTLQDDYSGSGFFDGFSFFT DTDPTNGFVDYVDEATAQSNGYISTSGDSVYMGVDHTNVA |
| | | GSSGRQSVRISSDATYNHGLFILDLEHMPGGICGTWPAFWL VGADWPNNGEIDIIEGVNQQSGNDMTLHTSDGC SISSSSDFTGSMTTDNCYVYAAGQSSNAGCGITDPDATSYG TAFNANGGGVFATEWTSDAISIWFFERGSIPDDIDSGNPDPD SWGSPVARFQGDCDIDSHFDGLQIIFDTTFCGDWAGNVWG SGSCASVASSCSSYVANNPGAFVDAYWSINSLK VYQDDGDSTVAGVVDYGDDDDEDEDDDQDDGDDDSDDD DDDDDDSDSDDDSDDDGDDDDDDDDDDDDDDEDSRPWRG GRNHHGQMLNASTSTSSFPSPSFVKKARWEPRNYMAGMIF |
| SEQ ID NO: 42 - *Aspergillus niger* | 5 | MKLSNALLTLASLALANVSTALPKASPAPSTSSSAASTSFAS TSGLQFTIDGETGYFAGTNSYWIGFLTDNADVDLVMGHLKS SGLKILRVWGFNDVTSQPSSGTVWYQLHQDGKSTINTGAD GLQRLDYVVSSAEQHDIKLIINFVNYWTDYGGMSAYVS AYGGSGETDFYTSDTMQSAYQTYIKTVVERYSNSSAVFAW ELANEPRCPSCDTSVLYNWIEKTSKFIKGLDADRMVCIGDEG FGLNIDSDGSYPYQFSEGLNFTMNLGIDTIDFGTLHLYPDSW GTSDDWGNGWITAHGAACKAAGKPCLLEEYGVTSN HCSVEGSWQKTALSTTGVGADLFWQYGDDLSTGKSPDDG NTIYYGTSDYQCLVTDHVAAIGSA |
| SEQ ID NO: 43 - *Aspergillus niger* | 26 | MFAKLSLLSLLFSSAALGASNQTLSYGNIDKSATPEARALLK YIQLQYGSHYISGQQDIDSWNWVEKNIGVAPAILGSDFTYYS PSAVAHGGKSHAVEDVIQHAGRNGINALVWHWYAPTCLLD TAKEPWYKGFYTEATCFNVSEAVNDHGNGTNYKLLLRDIDAI AAQIKRLDQAKVPILFRPLHEPEGGWFWWGAQGPAPFKKL WDILYDRITRYHNLHNMVWVCNTADPAWYPGNDKCDIATID HYPAVGDHGVAADQYKKLQTLTNNERVLAMAEVGPIPDPDK QASENVNWAYWMVWSGDFIEDGKQNPNQFLHKVYNDTRV VALNWEGA |
| SEQ ID NO: 44 - *Aspergillus niger* | 17 | MLSKMQLAQLAAFAMTLATSEAAYQGFNYGNKFSDESSKF QADFEAEFKAAKNLVGTSGFTSARLYTMIQAYSTSDVIEAIPA AIAQDTSLLLGLWASGGGMDNEITALKTAISQYGEELGKLVV GISVGSEDLYRNSVEGAEADAGVGVNPDELVEYIKEVRSVIA GTALADVSIGHVDTWDSWTNSSNSAVVEAVDWLGFDGYPF FQSSMANSIDNAKTLFEESVAKTKAVAGDKEVWITETGWPV SGDSQGDAVASIANAKTFWDEVGCPLFGNVNTWWYILQDA SPTTPNPSFGIVGSTLSTTPLFDLSCKNSTTSSSSAVVSAAA SSAAGSKAVGSSQASSGAAAWATSASGSAKPTFTVGRPGV NGTVFGNGTYPLRPSGSASARPSAGAISSGSGSSSSGSGS SGSTGTSATSGQSSSSGSSAAAGSSSPAAFSGASTLSGSLF GAVVAVFMTLAAL |
| SEQ ID NO: 45 - *Aspergillus Niger* | 5 | MKTLSLAAVLLVQAWTANSSPRAKRSSGFQWFGSNESGAE FGSGNIPGTLGTDYIWPDATAIATLHSAGMNIFRVPFLMERL VPDELTGAADATYMADLKATVQTITDLGAYAVVDPHNFGRY YGNIITSTSDFAAFWTTVASEFTDNDLVIFDTNNE FHDEDQTLVLDLNQAAITAIRATGATTQYIFVEGNSYTGAWT WTTTNTNLVNLTDPNDHLIYEMHQYLDSDGSGTSSTCVSSTI GAERIADATTWLKENGKQAVLGEFAGGANSVCEEAVTGML DAMVDASDVWLGGMWWSAGPWWGDYIFS LEPDDGIAYEYYLGVLEGYTPGSSSGSSGGSTTSAVASASS TSSAGSTTSKVTTTTAATATSTAVAAHYAQCGGEGWTGATT CADGYTCTVENEWYSQCL |
| SEQ ID NO: 46 - *Aspergillus Niger* | 12 | MKVLALSALLSLASAASISRRSDFCGQWDTATAGDFILYNDL WGEDNASSGSQCTGVDSASGSEIAWHTSWSWEGGSSDVK SYANAALQFTGTQLSSISSIPSTWKWTYSGSDIVADVAYDMF LGSTADASSDEYEIMVWLAALGGAGPISSTGSTIATPTINGVT WDLYTGPNGDTTVYSFVAQSTTEDFSGDLNDFFTYLVDNE GVSDSLYLTTLEAGTEPFTGSDAELKVSEYSVSIE |
| SEQ ID NO: 47 - *Aspergillus Niger* | 12 | MNRYLSIPLCLASAVPALAGLAARDPIGTICTKNNIITTDDFILY NNLWGEDYATSGSECTYLDYDSGNSISWQTSWTWAGGDD YVKSYPNAVLNVGAKQLSTITSIPSTWKWSYTGNDLVADVS YDAFLSTTDSTTATHEYEIMIWLAAYGGIEPIGNSDGPIASPTI GGYTWDLYKGPNDWTVYSFVARETITDFSADVLEFFTYLVD NEGVSSSLYLQTLGAGTEPKTGSDAWFTVSPYTVSINT |
| SEQ ID NO: 48 - *Aspergillus Niger* | 5 | MRISNLIVAASAASMVSALPSRQMKKRDSGFKWVGTSESGA EFGSALPGTLGTDYTWPETSKIQVLRNKGMNIFRIPFLMERL TPDGLTSSFASTYLSDLKSTVEFVTNSGAYAVLDPHNYGRF DGSIITSTSDFKTWWKNVATEFADNDKVIFDTNNE YHDMEQSLVLDLNQAAINGIRAAGATTQYIFVEGNAYTGAW DWTTYNDNLSGLTDSEDKIIYEMHQYLDSDSSGTSETCVSS TIGQERLEKATEWLKTNNKQGIVGEFAGGVNSVCEEAVEGM LAYMSENSDVWVGASWWSAGPWWGTYMYSLEPTDGTAY STYLPILEKYFPSGDASASSSASVSVAAATSTASTTTAAFEQ TTTPATQVEIASSSSSSSANAASQTTLSKVKSKSKSPCKLSS ATSSAVAATTPVVVATTPAVAPTSSSVAFVTTSVYVPTTTAA VAPSQVSSSAAASSSGVVGVSDPQGPSATNSAGEVNQYYQ CGGINWTGPTVCASPYTCKVQNDYYYQCVAE |
| SEQ ID NO: 49 - *Aspergillus Niger* | 5 | MKFQSTLLLAAAAGSALAVPHGSGHKKRASVFEWFGSNES GAEFGTNIPGVWGTDYIFPDPSTISTLIGKGMNFFRVQFMME RLLPDSMTGSYDEEYLANLTTVVKAVTDGGAHALIDPHNYG RYNGEIISSTSDFQTFWQNLAGQYKDNDLVMFDTN |
| | | NEYYDMDQDLVLNLNQAAINGIRAAGASQYIFVEGNSWTGA WTWVDVNDNMKNLTDPEDKIVYEMHQYLDSDGSGTSETCV SGTIGKERITDATQWLKDNKKVGFIGEYAGGSNDVCRSAVS GMLEYMANNTDVWKGASWWAAGPWWGDYIFSLEPPDGT AYTGMLDILETYL |

### Strain Cultivation for Sequencing

*Trichoderma reesei* - *480 (CBS 144197)* was cultured in PDA slants at 30°C for 10 days. After the incubation period, the microbial layer formed in the slants was used to inoculate 500 ml flasks (working volume of 125 ml) containing YEG media. The flasks were incubated at 30°C at 220 rpm until enough biomass was produced. Using sterile vacuum filter units, the biomass was carefully harvested and collected in sterile 50 mL centrifuge tubes. The biomass was immediately snap frozen using liquid nitrogen and stored at -80°C until further analysis.

*Aspergillus niger* - 277 *(no deposit number)* was cultured in wheat bran slants at 30°C for 10 days. After the incubation period, the microbial layer formed in the slants was used to inoculate 500 ml flasks (working volume of 125 ml) containing YEG media. The flasks were incubated at 30°C at 220 rpm until enough biomass was produced. Using sterile vacuum filter units, the biomass was carefully harvested and collected in sterile 50 mL centrifuge tubes. The biomass was immediately snap frozen using liquid nitrogen and stored at -80°C until further analysis.

*Rasamsonia composticola* - *427 (CBS 133861)* was cultured in PDA slants at 44°C for 10 days. After the incubation period, the microbial layer formed in the slants was used to inoculate 500 ml flasks (working volume of 125 ml) containing YEG media. The flasks were incubated at 44°C at 220 rpm until enough biomass was produced. Using sterile vacuum filter units, the biomass was carefully harvested and collected in sterile 50 mL centrifuge tubes. The biomass was immediately snap frozen using liquid nitrogen and stored at -80°C until further analysis.

The frozen cell pellets of all three strains were used for DNA extraction.

### Next Generation Sequencing

DNA extraction was performed using a phenol-chloroform protocol, suitable for extraction of both genomic and episomal plasmid DNA. The DNA extracted was used to prepare Nextera Flex (Illumina) and PacBio (Pacific Biosciences) sequencing libraries. Both libraries were then used to obtain the final genome assembly used for downstream analyses, that is, functional annotation, where predicted genes were annotated with biological functions.

### Genome Assembly

A de novo genome assembly was obtained using PacBio long reads and the HGAP program in SMRT Link v9. The HGAP algorithm includes three steps: preassembly, de novo assembly, and assembly polishing. Next, Illumina reads were aligned to the HGAP assembly and unaligned reads were assembled de novo using SPAdes version 3.10. The order of contigs and the distances between them were estimated using the insert size information derived from an alignment of the paired-end reads to the draft assembly. Consequently, contigs were linked together and placed into scaffolds using SSPACE version 2.3. Using Illumina reads, gapped regions within scaffolds were (partially) closed using GapFiller version 1.10. Finally, assembly errors and the nucleotide disagreements between the Illumina reads and scaffold sequences were corrected using Pilon version 1.21. If present, scaffolds >1.5 kb were added to the HGAP assembly to obtain the final genome assembly used for all downstream analyses.

The eukaryotic genome assembly was annotated using BaseClear's Eukaryotic Genome Annotation pipeline that consists of two main parts: structural annotation, where eukaryotic gene prediction is performed using the AUGUSTUS software, and functional annotation, where predicted genes are annotated with biological functions. For functional annotation, the principles of the Prokka software with options appropriate for eukaryotic genomes are used. The pipeline includes a number of features:
- Eukaryotic gene prediction by Augustus using a model organism;
- tRNAs prediction by Aragorn version 1.2.38;
- pCDS physic-chemical properties using an in-house script;
- Inferred proteins: - EC number from UniProt BLAST best hit (version downloaded 2019-10) - Signal peptide from SignalP version 4.1 - Cellular localization from SignalP version 4.1 - Function annotation from BLAST UniProt best hit (version downloaded 2019-10) - Conserved domains by HMMER-3 (version downloaded 2019-10).

To assess the quality of the genome annotation, the BUSCO (Benchmarking Universal Single-Copy Orthologs) tool was used, with the gene set (proteins) mode of BUSCO (version 3.0.2) and the Eurotiomycetes ODB9 lineage database.

### Example 3

### Definition of Thermology

**Single enzyme preparation (SEP).** An enzyme preparation which has been produces by a single organism. Each single enzyme preparation possesses two or three different enzyme activities. Example: A single enzyme preparation produced by *T. reesei* contains xylanase, cellulase and beta-glucanase activity.

**Multi-component enzyme preparation (MCP).** A combination of every single enzyme preparations. Example: A combination of SEP's produced by *T. reesei, A. niger and R. composticola.*

**Main activity.** Predominant enzyme activity of a SEP from a certain production organism. Example: The main activity of *R. composticola* is considered to be beta-glucanase.

**Side activity.** Enzyme activity which is not predominant of a certain production organism. Example: The side activity of *R. composticola* is considered to be xylanase.

**Total activity.** Main and side activity of a certain enzyme activity in a MCP.

Example: In a MCP, the total activity of beta-glucanase is the sum of the main activity from R. *composticola* and the side activity from *T. reesei, A. niger.*

An overview about the main and side activity of each production organism is given in Table 1.

**Table. 1. Main and side activity of production organisms.**

| **Organism** | **Xylanase** | **Cellulase** | **Beta-Glucanase** |
|---|---|---|---|
| *Trichoderma reesei* | SA | MA | SA |
| *Aspergillus niger* | MA | SA | SA |
| *Rasamsonia composticola* | SA | SA | MA |

| | | | |
|---|---|---|---|
| MA = main activity; SA = Side activity | | | |

Bake tests were carried out in two phases. In the first phase, a single enzyme preparation (SEP) has been applied to investigate the effect on dough handling / rheology and on rye bread quality. Different dosages were tested in order to investigate the optimal range of enzyme activity applied per kilogram of flour, which shows the desired effect. Single enzyme preparations have been combined to a multi-component enzyme preparation (MCP) in order to optimize their technical performance and investigate a synergy effect in the production of rye breads. This was performed in the second phase of the project.

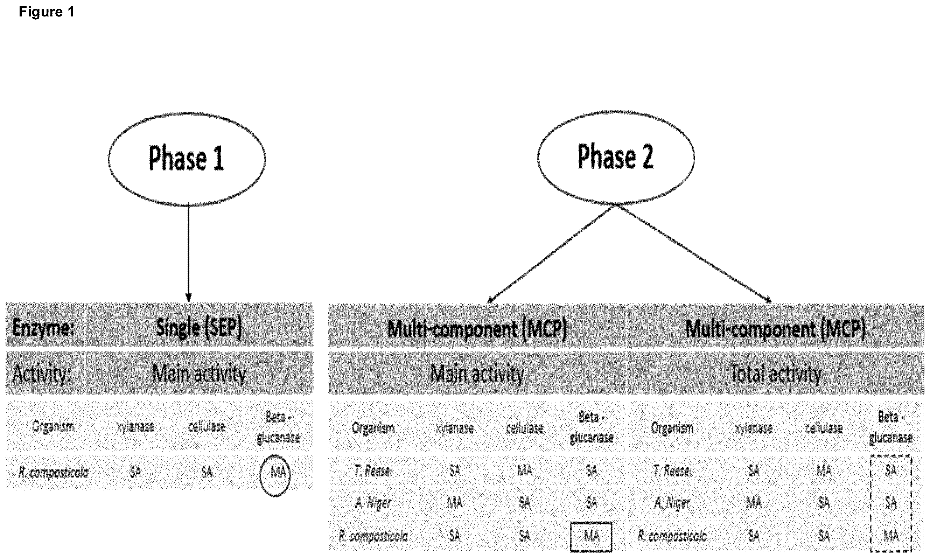

An overview of the experimental design of both phases is given in Figure 1, where "MA" indicates main activity and "SA" indicates side activity.

### Example 4

### Bake Trial with 70% rye flour in formulation

All raw materials being used for the trials came from the same batch code with a good best before date. Flour, sourdough, salt and water were all stored at the same ambient temperature. The enzyme solution and compressed yeast were stored in the refrigerator. Flour temperature was recorded on each trial day to calculate water temperature in order to achieve desired dough temperature. A kettle was used to achieve the desired water temperature which was higher than room temperature. Desired dough temperature for this Rye Bread application is 26 to 28°C. Desired pH for this Rye Bread application is 4.6.

**Flour specification:**

| | | |
|---|---|---|
| **Wheat Flour** | Moisture (%) | 15.5 |
| | Protein Content (%) | 11.5 |
| | Ash (%) | 0.6 |
| | Falling Number (sec) | 260 |
| | Farinograph Water Absorption (%) | 59 |
| **Rye Flour** | Moisture (%) | 14.5 |
| | Ash (%) | 0.88 |
| | Falling Number (sec) | 180 |

**Composition of the base dough in the test bakes was as follows:**

| ***Ingredients*** | % | ***Additional Information*** |
|---|---|---|
| Wheat Flour | 30 | Purchased from Meneba, Netherlands (Art.nr. 2821) |
| Rye Flour | 70 | Purchased from Meneba, Netherlands (Art.nr. 3530) |
| Dried Sourdough | 4 | Purchased from Boecker, Germany (Art.no. 84000200), calculated on total flour weight |
| Salt | 1.2 | Standard, calculated on total flour weight |
| Yeast | 2 | Compressed Baker's Yeast, calculated on total flour weight |
| Water | 75 | 26 to 27°C (calculated by desired dough temperature X2 - 1°C (temperature increase during mixing) - Flour temperature (recorded on the day). 75% calculated on total flour weight |

A 10.9kg batch of dough was prepared for each trial. All dry raw materials were scaled and added to the spiral mixer bowl. The enzyme solution was added to the bowl with the flour. Enzymes dosages used in the first and second phase are summarized in Table. 2.

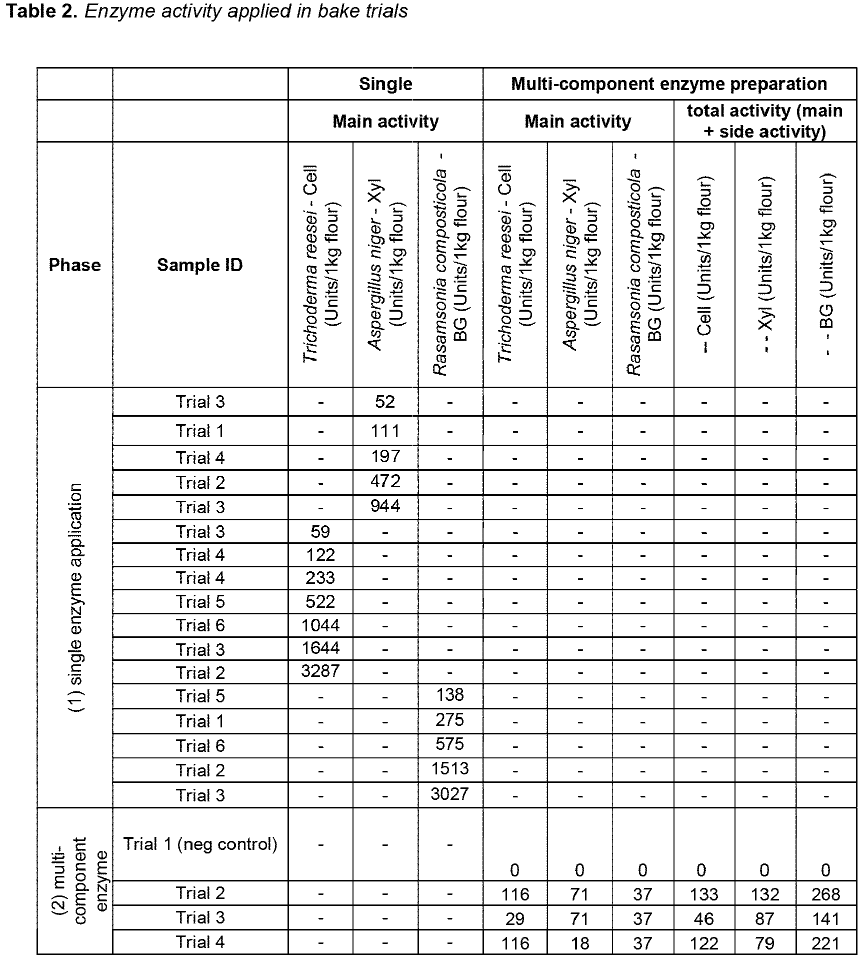

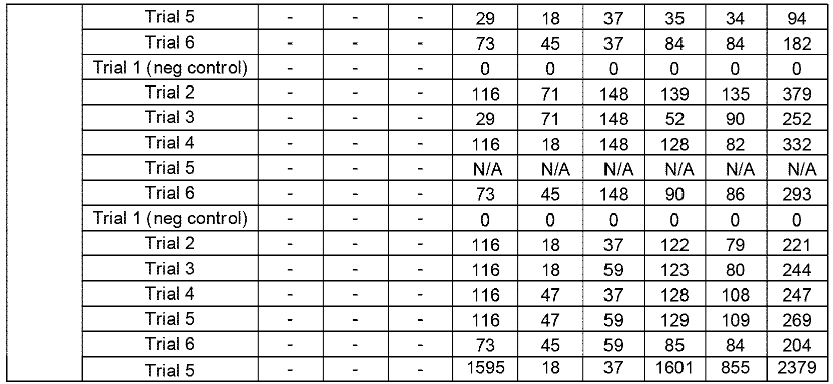

To make the dough, ingredients were mixed in 'Kemper' Spiral mixer on slow speed for 1000 turns/revolutions (approx. 6.6 minutes depending on mixer). Dough was then removed from the bowl and placed on a floured tray. Temperature and pH of the dough were recorded at this point. Dough firmness and stickiness also assessed at this point and rated as following.

| | |
|---|---|
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not Acceptable | Very sticky dough, lack of dough stability- machinability and cleaning issues very likely |

Dough was then covered down with a Couche cloth (to prevent skinning) and left to rest for 20 minutes. Dough was scaled at 1000g and moulded round. After 2 minutes rest, the dough was then shaped accordingly. Four of the dough samples were shaped and placed into Loaf tins; the remaining six dough pieces were shaped and placed into floured proving baskets with seam side up. All samples were gently pressed down by hand before entering the proving cabinet. Dough was proved for 45min at 38°C and 80% relative humidity. Dough samples in the tin were docked on the surface using the pastry dock and loaded into the ovens. Three of the dough samples in proving baskets were gently tipped out onto the loading belt, docked with pastry dock on the surface and loaded into the oven to be baked on the oven floor. The remaining three samples from the proving baskets were subjected to a 'shock' test in order to assess dough stability. This was achieved by gently tipping the dough out onto a tray lined with baking parchment and docking the surface using the pastry dock. This tray was then dropped from a height of 40cm before loading samples immediately into the oven to be baked on the oven floor.

All trials were baked in Wachtel deck ovens. Top temperature of 260°C, bottom temperature of 250°C for 1 minute, dropping temperature to 200°C top and bottom for the remaining 59 minutes. 5 seconds of steam added during the first minute of baking. Breads cooled for 2 hours before being packed separately into plastic bags and labelled accordingly.

Following equipment was used: Kemper Spiral Mixer, Couche Cloth, 800g Baking Tins (Bread-Loaf), Proving Baskets (Oval), Temperature Probe, pH Probe, Temperature and Humidity controlled proving cabinet, Pastry Docker, Wachtel Deck Oven, Bread Slicer, TA Micro Systems, Volscan Profiler, Plastic Zip Lock Bags.

### Example 5

### Post Bake Analysis

In order to assess the technical performance of the enzyme in rye bread manufacturing, specific bread volume (Volscan), softness (texture analyser) and baker's touch were carried out post baking. The Volscan Profiler is a benchtop laser-based scanner that measures the volume, density and dimensional profiles of solid products. The following settings were used for measurements:

| | |
|---|---|
| **Vertical Step** | 2.0mm |
| **Rotation Speed** | Fast 1.5rps |
| **Product Weight** | Entered manually |

From each trial, 2 tin loaf samples, 3 basket loaf samples and 3 shocked basket loaf samples were selected for Volscan. Specific Volume, Width at max height and Height to max Width ratio (basket breads) were recorded and results presented in a graph. Tin loaf was compared with tin loaf, Basket loaf compared with basket loaf, shocked basket loaf compared with non-shocked basket loaf.

Texture Analyser - The TA.XTplus was fitted with a 5kg load cell and 1" diameter cylinder probe (P/1) probe before carrying out our test. The method selected: AIBCAKE1/P1. This TPA (Texture Profile Analysis) test compresses the sample to a fixed distance, withdraws to the original sample height as determined by the trigger force, allows the sample to rest/recover for a fixed time period, and compresses to precisely the original penetration distance. Based on the products behaviour, hardness (or softness), springiness, cohesiveness, gumminess (valid only for semi-solid products), chewiness, fracturability and resilience can be quantified. This study focuses on hardness and springiness.

| | | |
|---|---|---|
| | Option; | TPA |
| | Pre-test speed: | 2.0mm/s |
| **TA Settings** | Test speed: | 2.0mm/s |
| | Post-test speed: | 2.0mm/s |
| | Distance: | 10mm |
| | Time: | 3s |
| | Trigger type: | Auto- 5g |
| | Tare mode: | Auto |
| | Data acquisition rate: | 200pps |

**Baker's Touch** - Samples from the tin loaves were also used to carry out 'Baker's Touch'. This analysis consisted of the trained bakers assessing the following attributes by sight, smell, touch and taste, as shown in Table 5, Table 6, Table 7, Table 9, Table 10, Table 12 and Table 14.

**Balling visible on the slice after slicing.** (i.e. were there little balls of crumb on the surface of the slice, if present are there more or less than the control or roughly the same); a positive number suggests a worse final result for this analysis.

**Crumb colour** (how does the colour of the crumb look compared to the control, is it lighter or darker or the same);

**Crumb porosity** (how open the cell structure looks compared to the control); a positive number suggests more open crumbs, which may not be considered detrimental together with an acceptable butterability.

**Butterability** (how quickly the slice crumbles when subject to rubbing with your finger- shows the strength of the crumb. Does it crumble faster or slower than the control or similar); a positive number suggests worse butterability.

**Softness to touch** (how does the slice feel compared to the control, is it softer or firmer or similar); a positive number suggests an improved softness.

**Aroma** (how the slice smells, does it differ from the control, if so in what way, descriptors used if necessary); a positive number suggests an improved aroma.

**Softness to bite** (is the slice softer or firmer to bite compared to the control or similar); a positive number suggests an improved softness.

**Moistness** (is the sample more or less moist when compared to the control or similar); a positive number suggests an improved softness.

**Cohesiveness** (when chewing the sample does it form a ball in the mouth faster than the control, does it crumble more and not form a ball, or is it similar to chew). a positive number suggests a worse perception as the sample is overly cohesive.

**Table 3. Baker's Touch Analysis**

| **Baker's Touch Attributes** | **Subjective / Objective** | **What does positive values mean?** |
|---|---|---|
| Processability | Subjective | N/ A |
| Height:Witdh ratio | Objective | N/ A |
| Sliceability | Subjective | N/A |
| Sensory (Flavour profile) | Subjective | Improved |
| Slicing / Balling | Subjective | Worse |
| Crumb colour | Subjective | N/A |
| Crumb resilience | Subjective | Improved |
| Aroma | Subjective | Improved |
| Cohesiveness | Subjective | Worse |
| Moistness | Subjective | Improved |
| Specific loaf volume (ml/g) | Objective | N/ A |
| Improved loaf volume (%) | Objective | N/ A |
| Specific Volume - Basket (ml/g) | Objective | N/ A |
| Improved bread volume - Basket (%) | Objective | N/ A |
| Softness Day+1 | Objective | N/A |
| Softness Day+5 | Objective | N A |
| Butterability | Subjective | Worse |
| Softness to touch | Subjective | Improved |
| Balling after slicing | Subjective | Worse |
| Softness to touch | Subjective | Improved |

All samples were photographed and presented in a comparison.

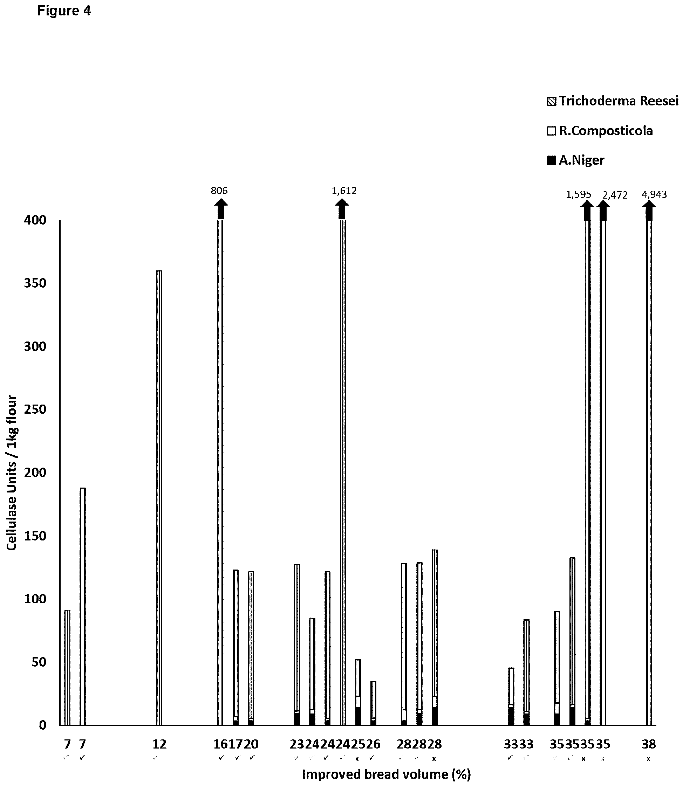

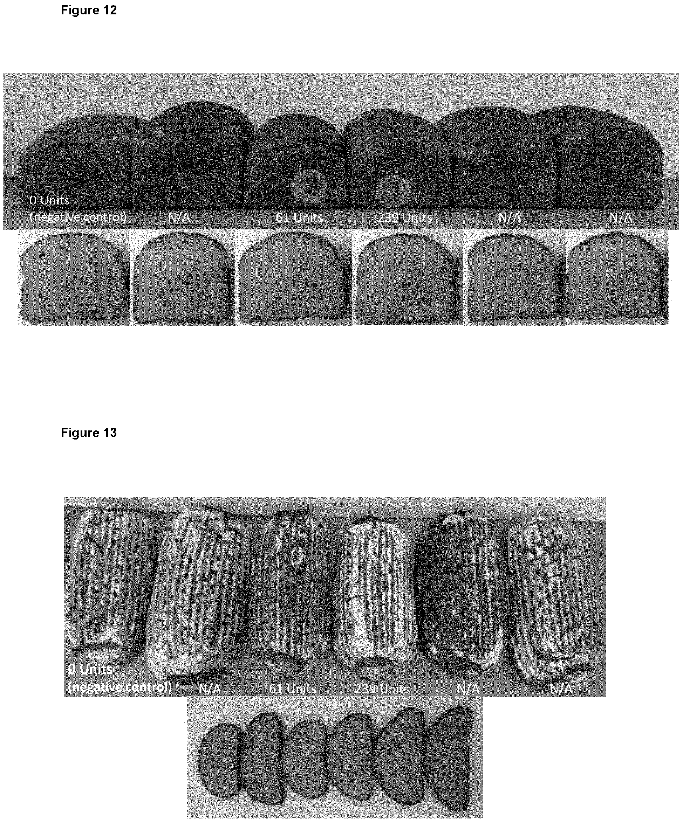

Figure 4 illustrates the effect of a cellulase applied as SEP from *T. reesei* on loaf bread characteristics. The application of 61 Units/1kg flour showed a small increase in bread volume and similar bread crumb characteristics, while 239 Units/1kg flour further improved brad volume and resulted in slightly more open crumb. Basket proofed breads maintained their shape (height to width ratio) and an increased bread volume with a slightly more open crumb porosity (Figure 13).

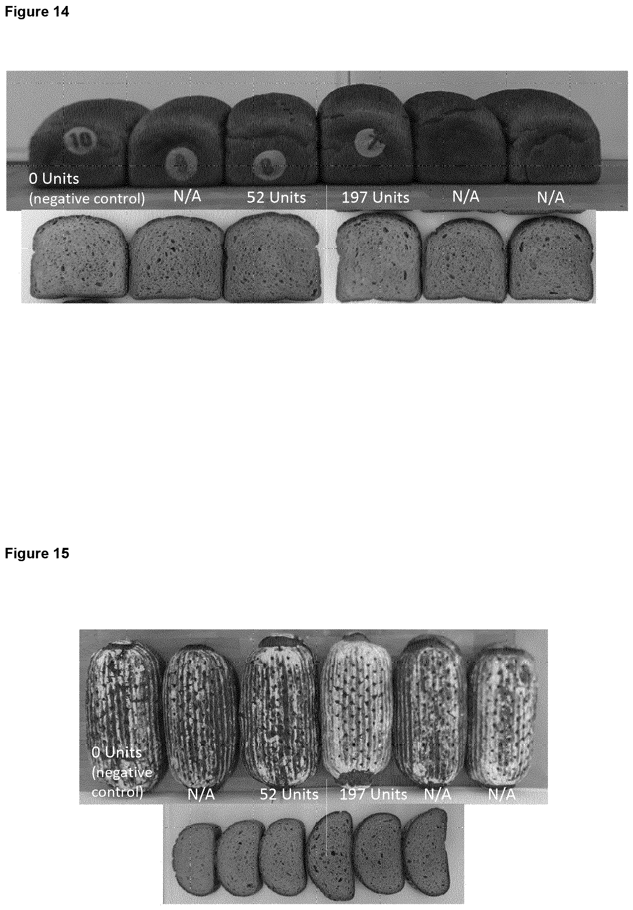

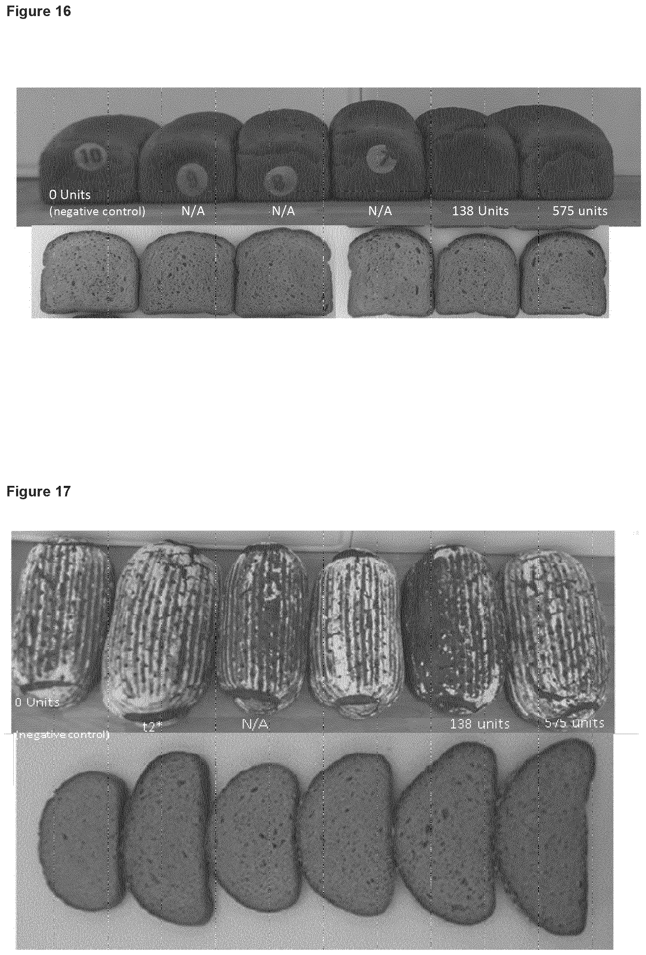

Figure 14 and 15 illustrate the effect of a xylanase applied as SEP from A. *niger.* Loafs and basket proofed breads showed am improved bread volume with a more open crumb structure. The addition of 197 Units/1kg flour resulted in drop of height to width ratio. Rye loafs with the application of beta-glucanase from *R. composticola* applied in a SEP were also higher in volume and more open in their crumb structure (Figure 16). The same observation was made for basked proofed breads, whereas the higher amount of 575 Units/1kg showed a decrease in the height to width ratio.

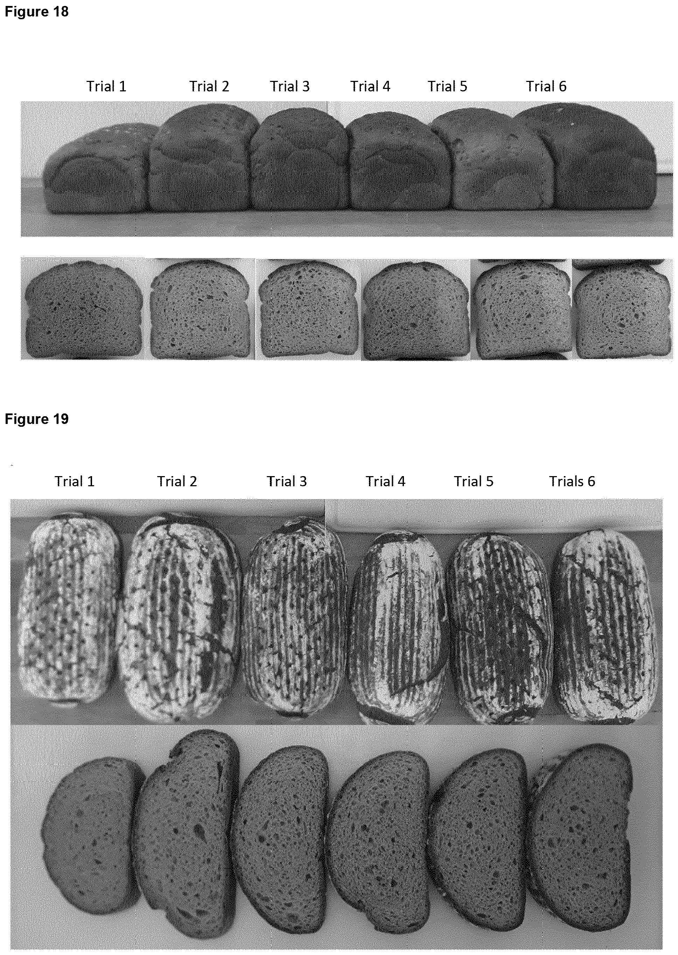

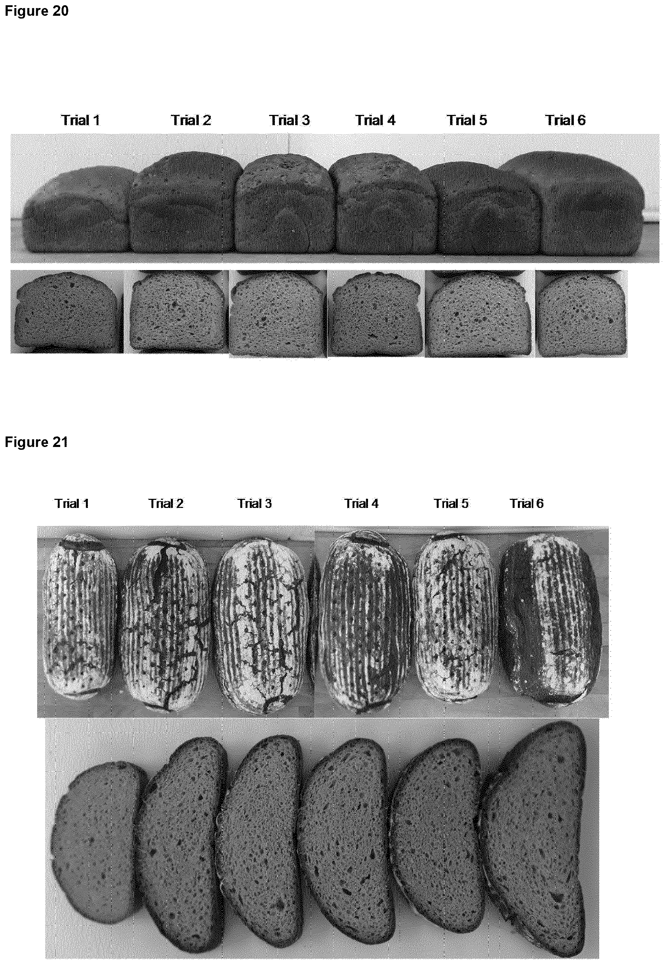

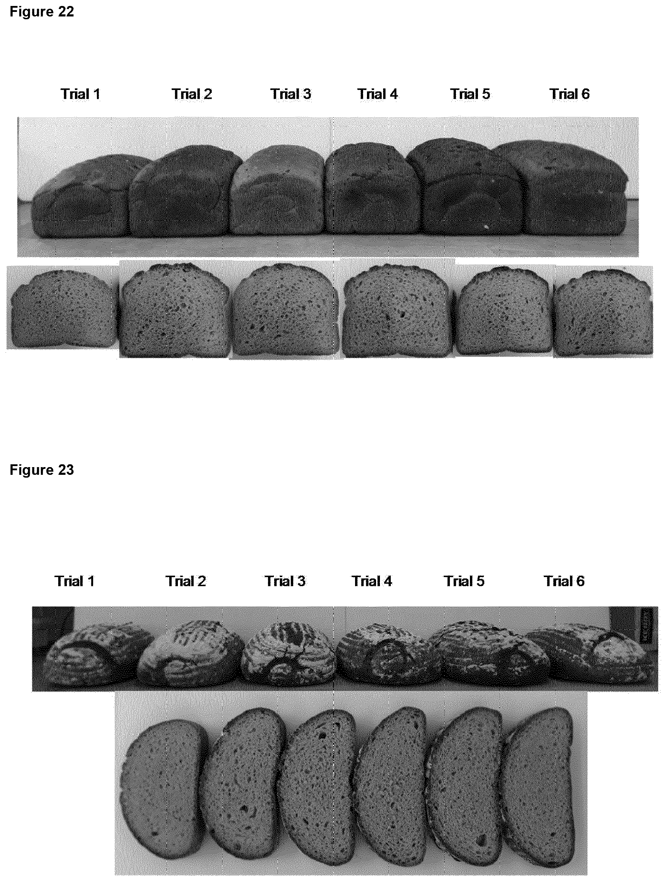

Figure 18 to 23 show the effect of a MCP in a rye bread formulation. The application of a MCP in rye loafs (Figure 18) and basked proofed breads (Figure 19) showed significant improved bread volume in all trials. All breads maintained a good/acceptable processability, showed a marginal drop of the height to width ratio and slightly more open crumb. In Figure 20, similar observations were made. Trial 2 and 3 (Figure 21) showed a drop in the height to width ratio, while all breads showed a significant improved volume. Loafs in figure 22 responded in a similar manner. All breads in Figure 23 showed improved bread volume, showed a marginal drop in height to width ratio, small increase in crumb porosity.

### Example 6

### In vitro characterization

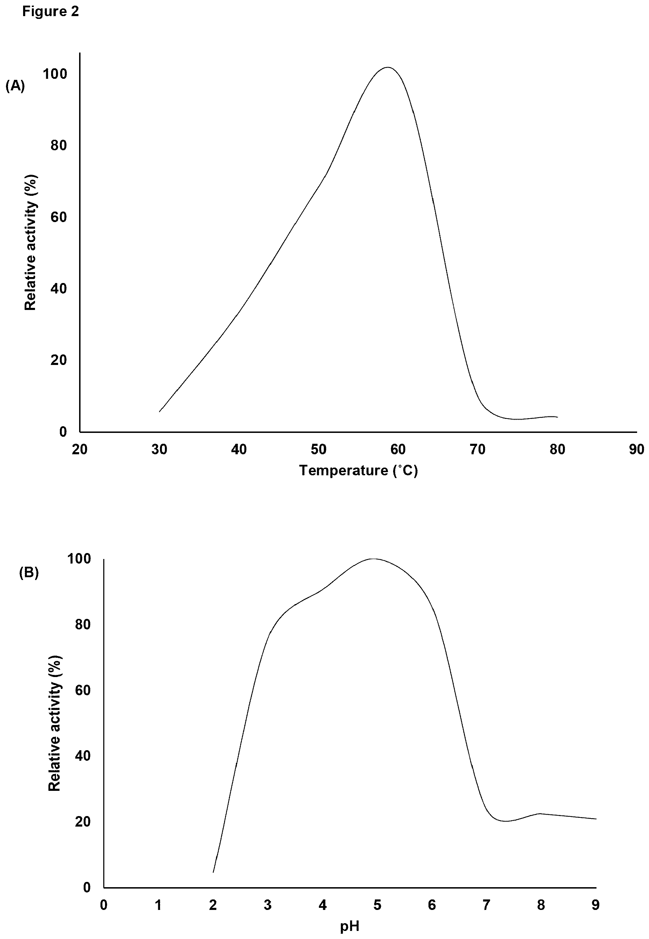

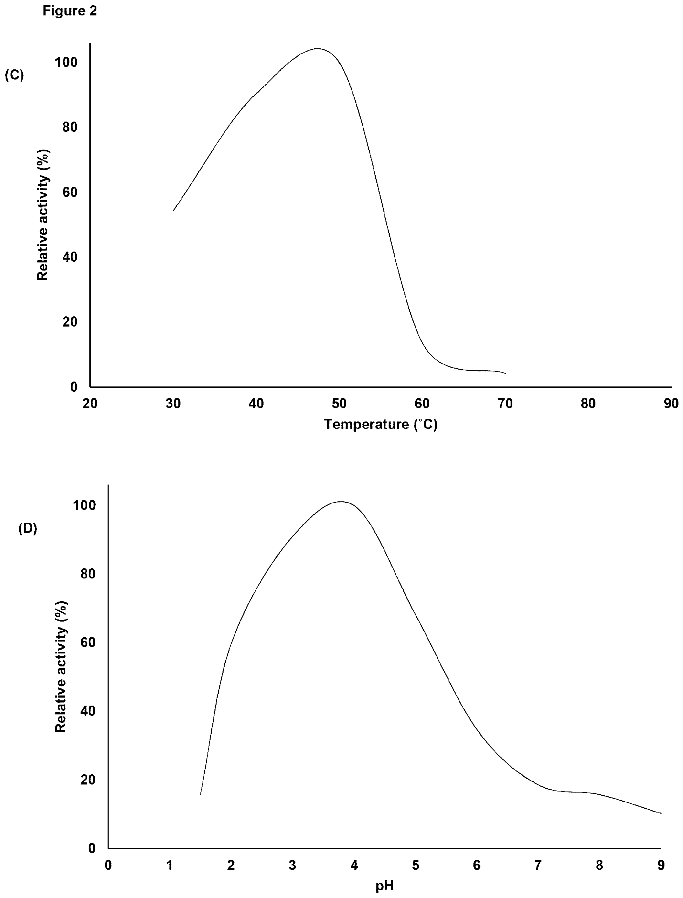

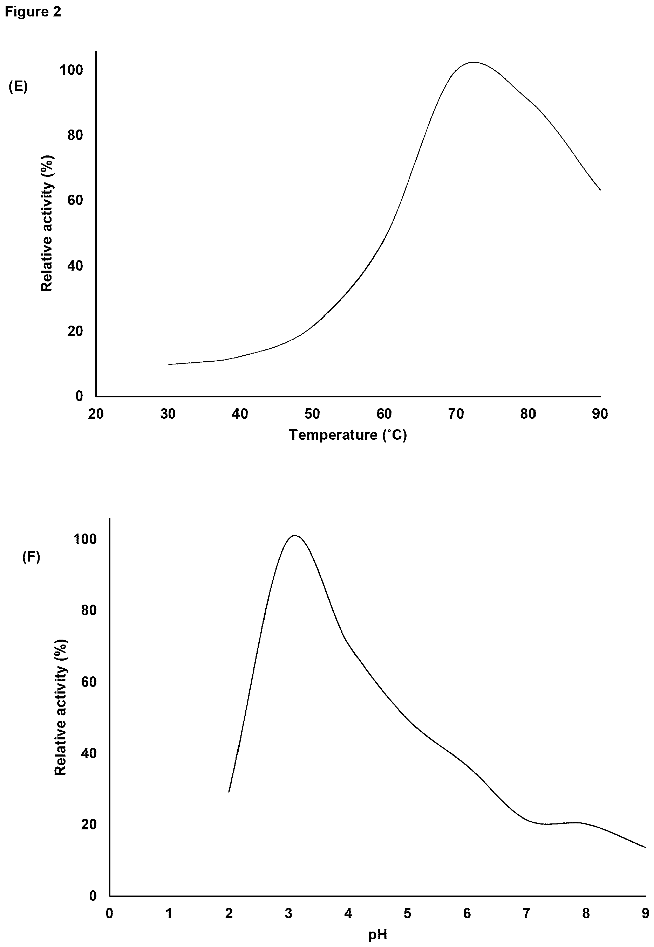

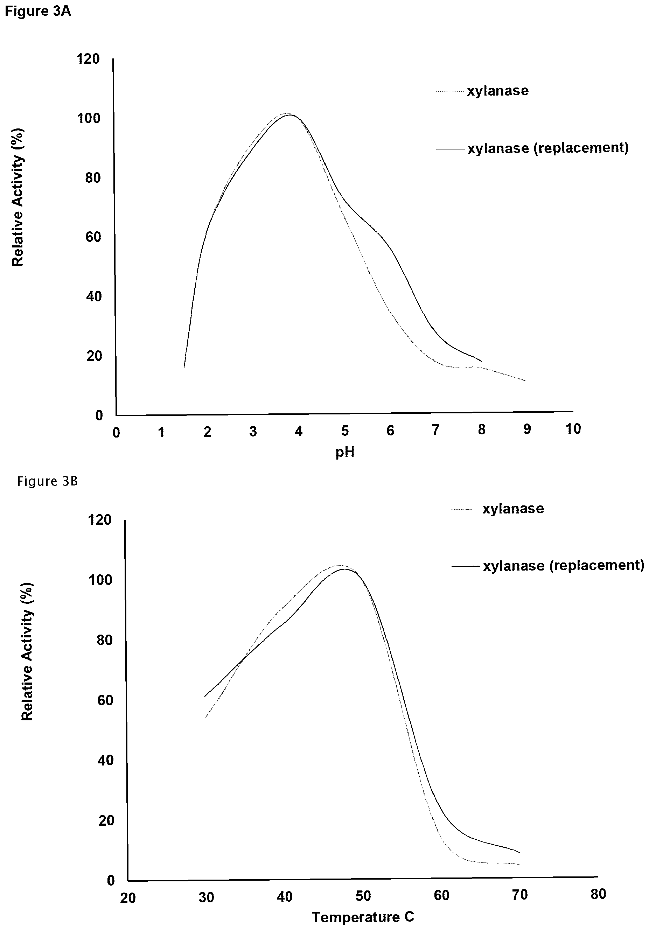

The pH and temperature profiles of cellulase, xylanase and beta-glucanase can be seen in Figure 2 and 3.

Table 4 describes the values (pH and temperature) where the enzyme activity drops below 50%. The lower and upper range describes the point of where the activity drops below this threshold. For cellulase the lower pH range is 2.6 and the upper is 6.6. The lower and upper limits of the temperature curve are 46°C and 65°C. Xylanase has a lower pH range of 2.3 and an upper of 5.5. The lower and upper limits of the temperature curve are <30°C and 56°C. Beta-glucanase has a lower pH range of 2.3 and an upper of 5.0. The lower and upper limits of the temperature curve are 60°C and >90°C.

**Table 4. Enzyme activity of 50% threshould**

| | | **Activity against pH** | | | | | | **Activity against Temperature** | | | | | |
|---|---|---|---|---|---|---|---|---|---|---|---|---|---|
| | | **Xyl** | | **Cell** | | **BG** | | **Xyl** | | **Cell** | | **BG** | |
| **Strain** | **Main activity** | *Lower* | *Upper* | *Lower* | *Upper* | *Lower* | *Upper* | *Lower* | *Upper* | *Lower* | *Upper* | *Lower* | *Upper* |
| *T Reesei* | cellulase | | | 2.6 | 66 | | | | | 46 | 65 | | |
| *A Niger* | xytanase | 2.3 | 5.5 | | | | | <30 | 56 | | | | |
| *R composticola* | beta-glucanase | | | | | 2.3 | 5.0 | | | | | 60 | 90 |

| | | | | | | | | | | | | | |
|---|---|---|---|---|---|---|---|---|---|---|---|---|---|
| Values state the range of pH and temperature (°C) where the enzyme activity is 50% or lower | | | | | | | | | | | | | |

### Example 7

### Bake Trials with 70% rye flour in formulation

The performance of single enzymes and a multi-component composition of non-starch polysaccharide (NSP) enzyme have been applied in rye bread processing. It has been found that an increased enzyme activity resulted in an increased volume. However, an over-dosage of enzymes may cause an excessive hydrolyzation of NSP, resulting in a slack and sticky dough. These detrimental effects of overdosing NSP enzymes have been reported previously. Therefore, a challenge for remains to provide an enzyme solution which delivers high bread volume while maintaining a good processability, which can be described as dough which possess a minimum of stickiness and no issues with machinability or cleaning could be expected.

Compared to a single enzyme application, the combination of enzymes showed an unexpected synergy in improving final product quality.

In particular, high increase in bread volume could be achieved while maintaining a good processability. Surprisingly, this superior effect was not observed and/or as distinct by applying a SEP.

It becomes evident by comparing the main activity of the same enzyme in the following examples. Applied in a MCP, 29 to 116 cellulase units resulted in an improved bread volume of 17 to 35%. On the other hand, 122 cellulase units improved bread volume by only 7% applied as SEP.

Figure 4 illustrates the amount of cellulase activity across all trials and reveals the dimensional differences. Significantly less cellulase activity, indicated by smaller bars, resulted in a higher bread volume, when cellulase was applied in a MCP.

As described earlier, an enzyme produced by different organisms may possess different properties. It would be therefore misleading to compare the total activity applied in a MCP against the main activity in the application of a SEP. Similar to cellulase, the usage of other enzymes showed similar results.

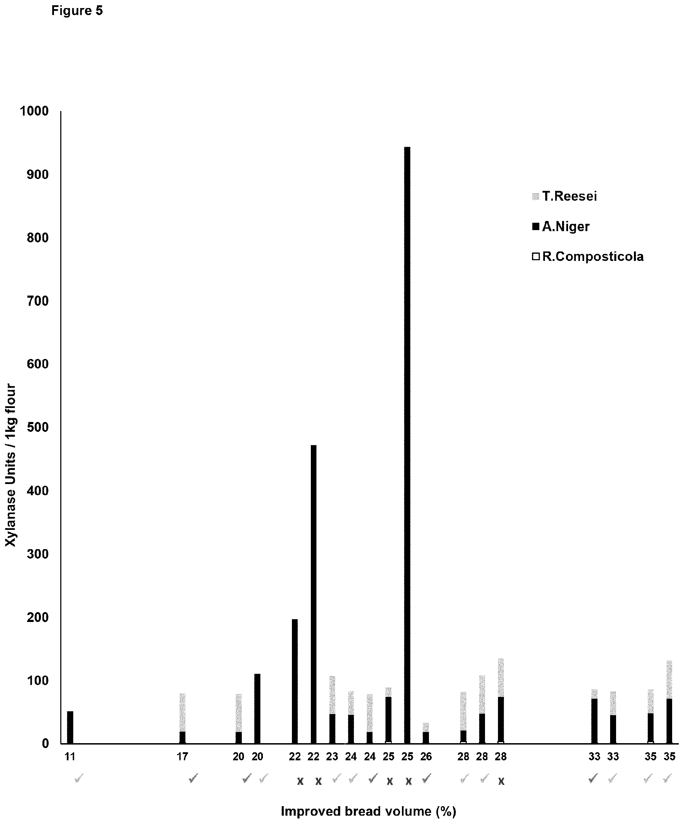

The dosage of 18 xylanase units as MCP increased bread volume by 17 to 28%. A higher amount of 52 to 111 xylanase units resulted in an improvement of only 11 to 20% in a SEP application. Figure 5 shows the xylanase proportions of all trials and shows that a small amount of xylanase resulted in a higher bread volume, when applied as a MCP.

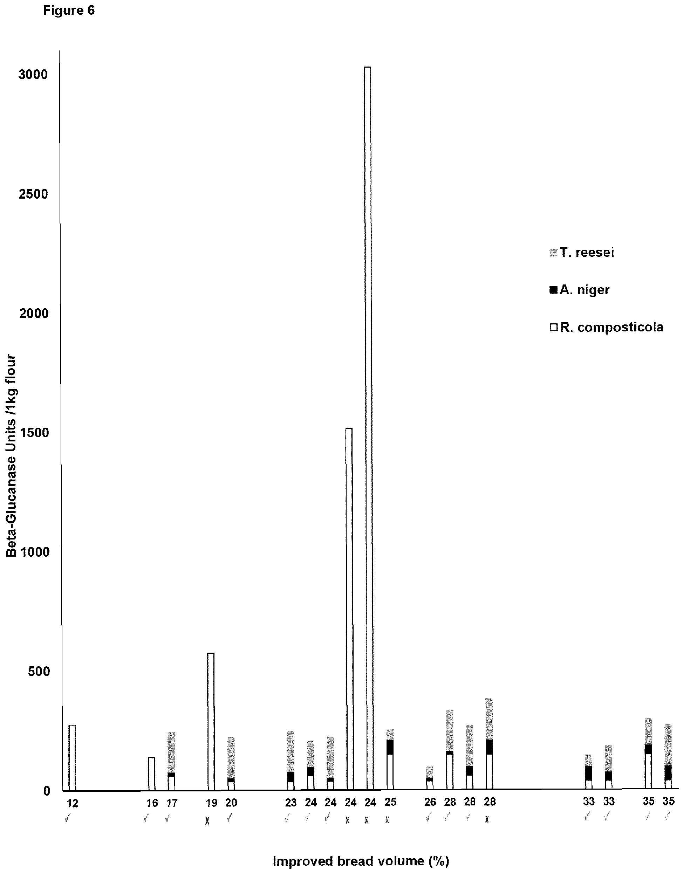

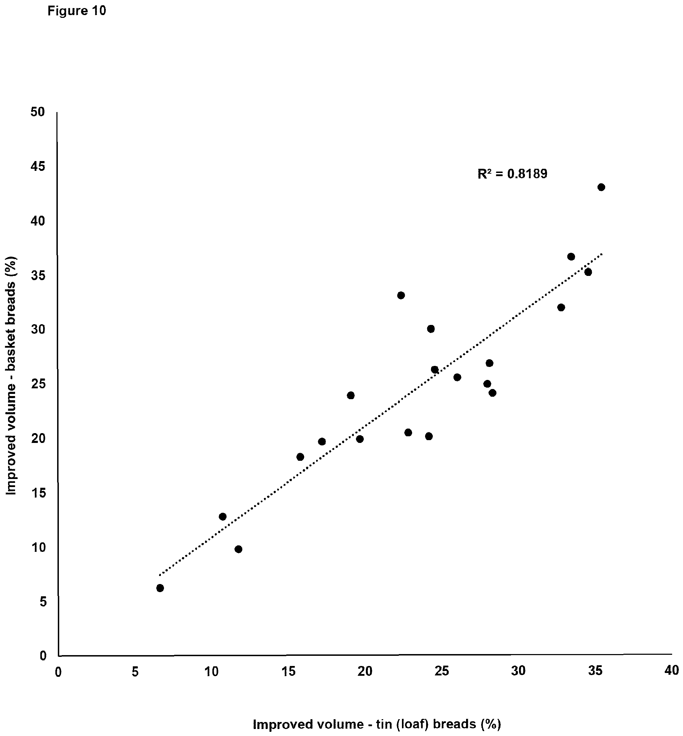

With 138 beta-glucanase units, loaf volume was improved by 16% in the application of a SEP. Compared to this, already 37 beta-glucanase units improved bread volume by 20 to 35% in a MCP application. The dimensional differences are illustrated in Figure 6, showing a marginal amount of beta-glucanase clearly exceeds the effect in loaf volume in the application of a MCP. A similar trend has been obtained when breads were proven in a basket indicated by a strong linear correlation of R² = 0.82 (Figure 10).

Breads produced with a MCP showed a good or acceptable processability. This was assessed by a skilled person of the art and further described by measuring a height to width ratio. It reflects the dough stability or its resistance against mechanical stress after proving, when breads are loaded in the oven. Acceptable values ranged between 0.5 to 0.6, whereas no enzyme addition resulted in value of approximately 0.6.

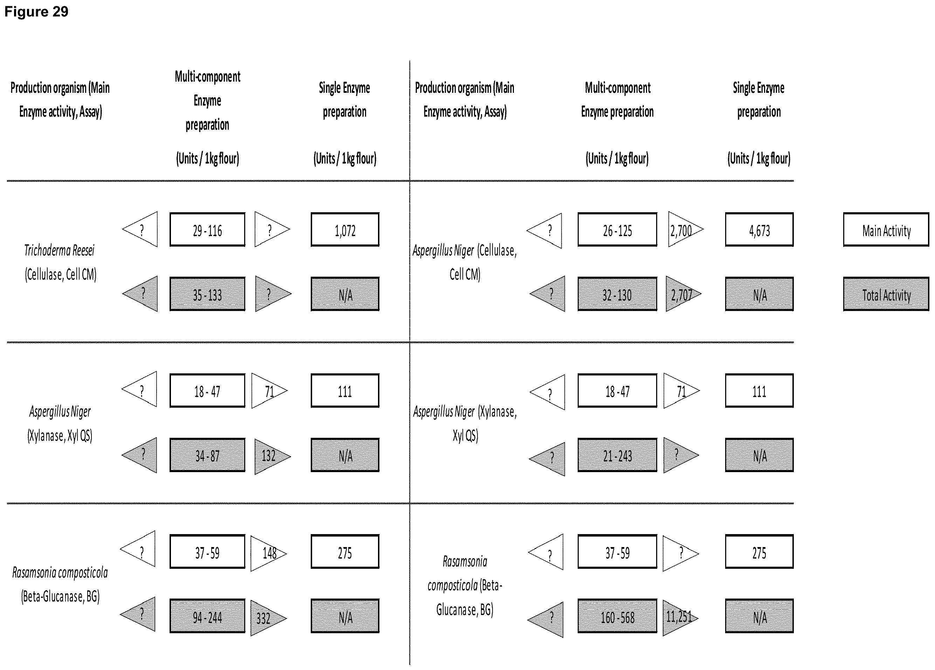

Figure 29 shows the range of enzyme activity for each enzyme (production organism) in a SEP and MCP, which showed promising results. A MCP may be prepared with a cellulase produced from a Trichoderma reesei or Aspergillus niger.

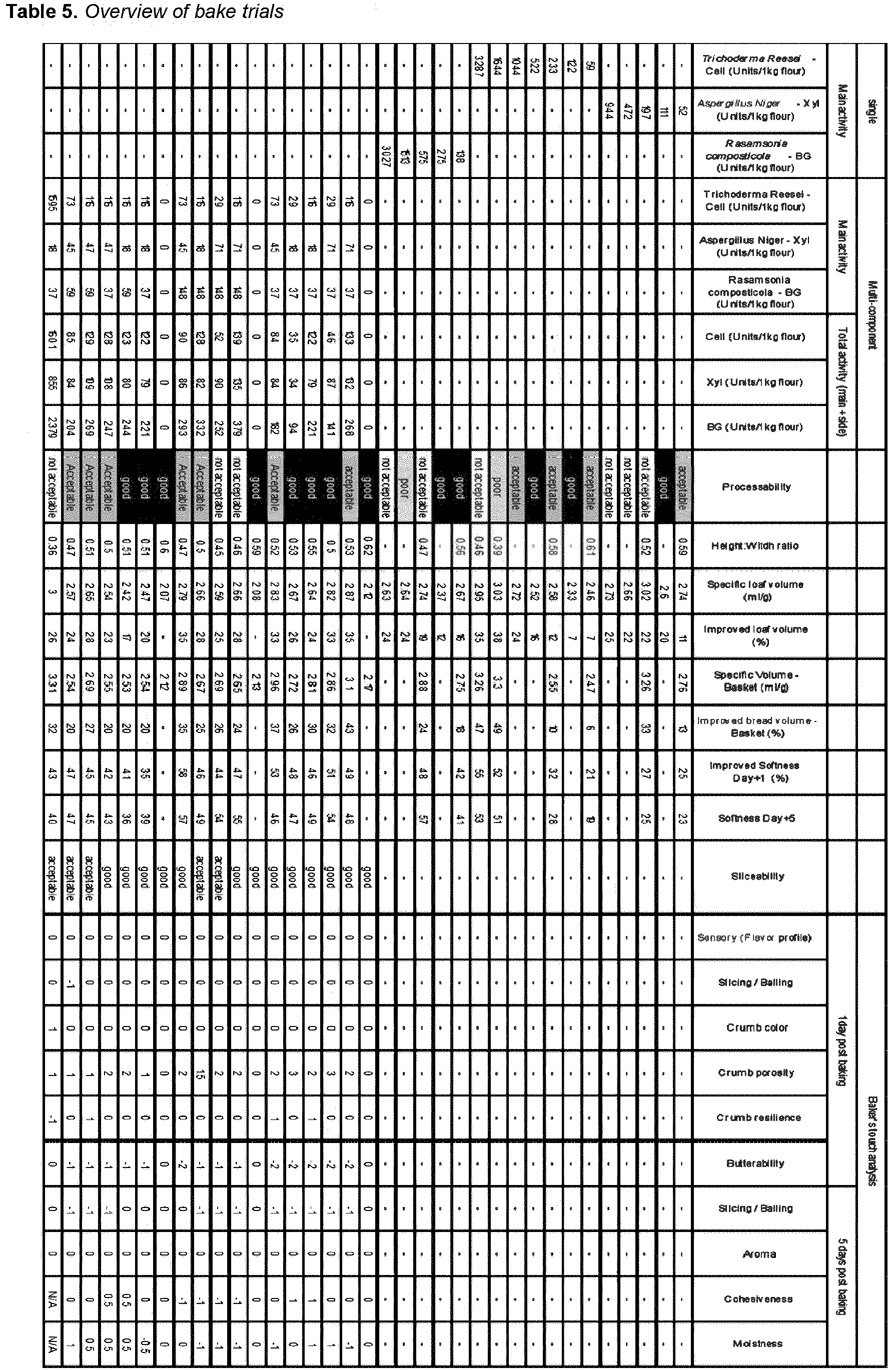

### Legend: Processability

| | |
|---|---|
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not acceptable | Very sticky dough, lack of dough stability- machinability and cleaning issues very likely |

Non-starch polysaccharide enzyme improved softness over shelf life as shown in Figure 29. Figure 29 also shows the range of enzyme activity for each enzyme (production organism) in a SEP and MCP, which showed promising results. A MCP may be prepared with a cellulase produced from a *Trichoderma reesei* or *Aspergillus niger.*

Table 5 results revealed a synergy effect when enzymes were combined.

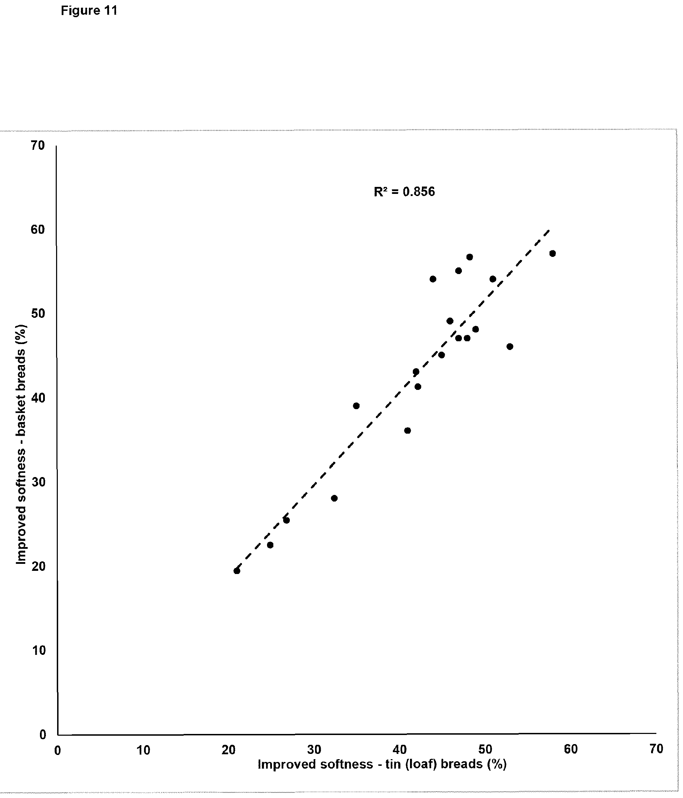

Applied as SEP, 59 cellulase units improved softness at day 5 by 19%, whereby 29 to 73 cellulase units improved softness of 47 to 57% applied as MCP. Across all enzymes, the results at day 1 showed a strong linear correlation of R² = 0.86 compared to day 5 (Figure 11).

The application of a SEP delivering 52 xylanase units improved softness by 23%, whereby a MCP application improved softness by 43 to 57% in the range of 47 to 71 xylanase units.

When 138 beta-glucanase units were applied as SEP, softness improved by 41%. Only 37 beta-glucanase units improved softness by 39 to 54% applied as MCP. A less firm crumb was observed in Baker's touch analysis one day post baking, whereas the differences were slightly more distinct five days post baking. Compared to a control, moisture and cohesiveness only showed minor differences.

The sensorial profile of bread is considered as key characteristic for every bread manufacturer. It is developed through the formulation of the recipe, production process and/or the usage of a sourdough. It is therefore mandatory to provide an enzyme solution, which does not impact the taste of the final product.

Across all trials, the addition of NSP enzymes did not have any impact on the flavour profile or the aroma of the final products. Crumb characteristics, such as balling, colour, resilience were also not negatively impacted. All trials showed larger crumb porosity, which is not considered to be detrimental for the final product quality and might be reasoned by a bigger expansion of the breads during baking. They also remained a good butterability with some breads scored lower than control, which was considered to be acceptable.

To validate the performance of certain enzymes in rye bread manufacturing, which possess similar specific properties, further bake tests have been conducted. For this, an alternative xylanase has been selected to test the performance in bake trials by replacing the xylanase previously tested.

Both xylanases are produced from an A. *niger* and show similar pH and temperature profiles with a slightly altering activity above pH 5 (Figure 3A). Trial 1 and 2 represent the control, which showed good performance in previous trials. Trial 3 and 4 are conducted with the alternative xylanase. No significant difference in rye bread characteristics has been obtained, when an equal amount of xylanase has been applied in MCP as indicated in Table 6. In a further trial, an alternative Cellulase was used to replace the cellulase previously tested. Both cellulases are produced from a *Trichoderma reesei* and possess similar pH and temperature profiles (Figure 3B).

No significant differences in dough properties or bread characteristics were obtained, when an equal amount of cellulase has been applied in MCP as indicated in Table 7. The alternative cellulase causes slightly more sticky, which was acceptable to process and showed a small increase in the width and a decrease in height: width ratio. No significant difference was found in improved loaf volume, while breads (basket proofed) showed a higher value of 4.7%. No difference in improved softness and baker's touch analysis was observed after 24 hours and 72 hours post baking. In both tests, the replacement of a cellulase and xylanase with similar pH and temperature profiles seems feasible when equal amounts of units are applied per 1kg flour.

Overall, the combination of three NSP enzymes resulted in an unexpected synergy in improving rye bread quality, when applied in a certain dose range and ratios between each other. Particularly, bread volumes and softness could be improved while a good processability was maintained. In addition, crumb characteristics and sensorial properties were not negatively affected, which emphasize the superior effect of a multi-component enzyme preparation in meeting the technical demand of bread manufacturer.

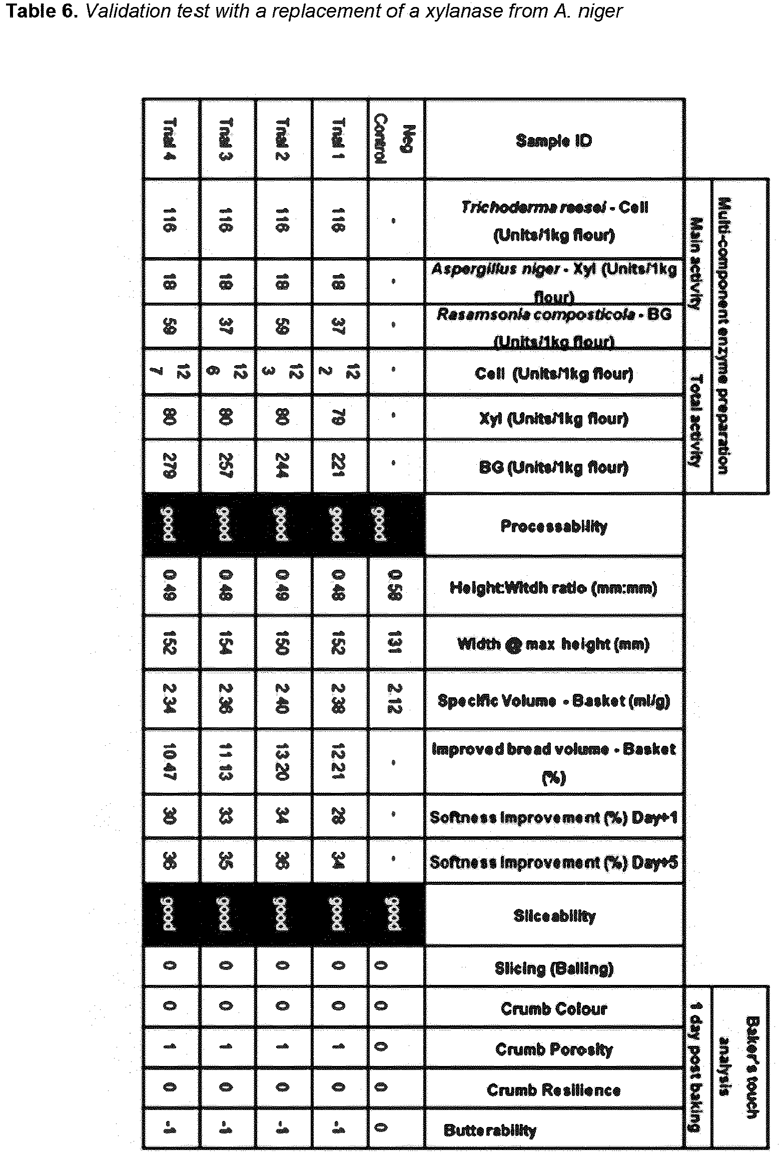

Trial 3 and 4 was conducted with an alternative xylanase.

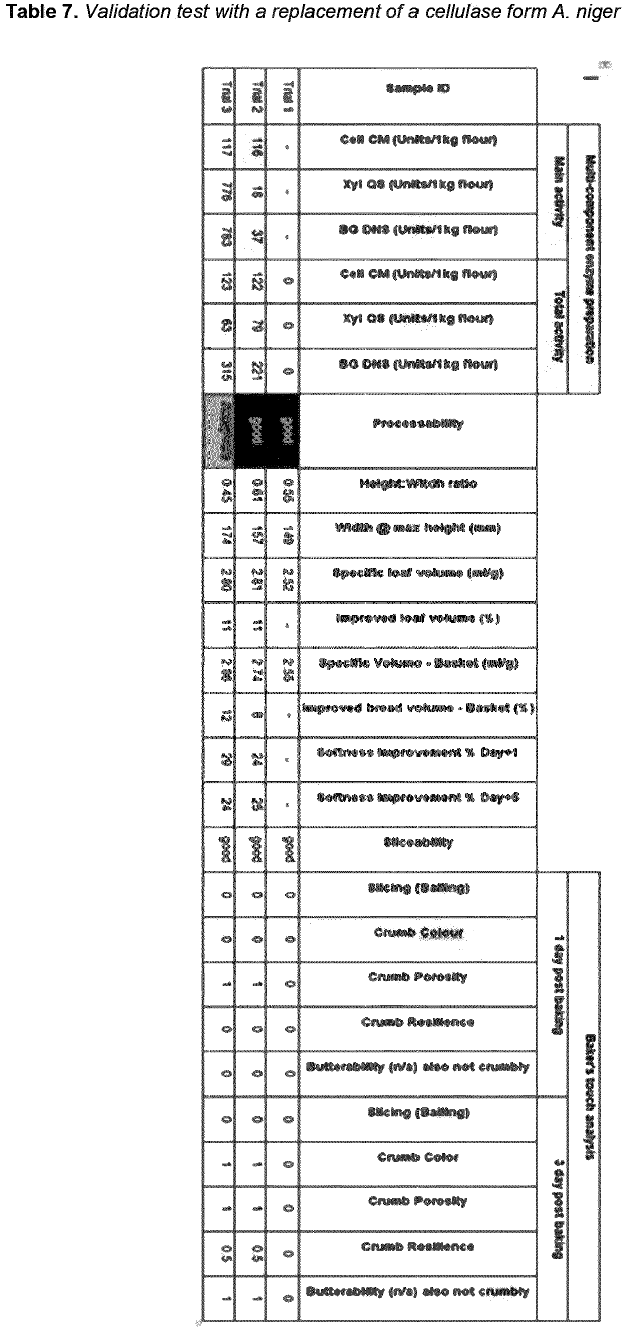

### Legend: Processability

| | |
|---|---|
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not acceptable | Very sticky dough, lack of dough stability- machinability and cleaning issues very likely |

### Example 8

### Performance in 20% rye flour formulation

The performance of a MCP of non-starch polysaccharide (NSP) enzymes have been tested in a recipe containing 20% rye flour.

Flour used in the test bakes possessed the following properties:

| | | |
|---|---|---|
| Wheat Flour | Moisture (%) | 15.5 |
| | Protein Content (%) | 11.5 |
| | Ash (%) | 0.6 |
| | Falling Number (sec) | 260 |
| | Farinograph Water Absorption (%) | 59 |
| Rye Flour | Moisture (%) | 14.5 |
| | Ash (%) | 0.88 |
| | Falling Number (sec) | 180 |

Composition of the base dough in the test bakes was as follows:

| **Ingredients** | **%** | **Additional Information** |
|---|---|---|
| Wheat Flour | 80 | Purchased from Meneba, Netherlands (Art.nr. 2821) |
| Rye Flour | 20 | Purchased from Meneba, Netherlands (Art.nr. 3530) |
| Ascorbic acid | 0.004 | Standard, calculated on total flour weight |
| Salt | 2 | Standard, calculated on total flour weight |
| Yeast | 3.5 | Compressed Baker's Yeast, calculated on total flour weight |
| Water | 61.5 | 25°C (calculated by desired dough temperature X2 - 1°C (temperature increase during mixing) - Flour temperature (recorded on the day). Calculated on total flour weight |

### Equipment used:

Kemper Spiral Mixer, Couche Cloth, 800g Baking Tins (Bread-Loaf), Proving Baskets (Oval), Temperature Probe, pH Probe, Temperature and Humidity controlled proving cabinet, Pastry Docker, Wachtel Deck Oven, Bread Slicer, TA Micro Systems, Volscan Profiler, Plastic Zip Lock Bags.

All raw materials being used for the trials came from the same batch code with a good best before date. Flour, ascorbic acid and salt were all stored at the same ambient temperature. The enzyme solution and compressed yeast were stored in the refrigerator. Flour temperature was recorded on each trial day to calculate water temperature in order to achieve desired dough temperature of approximate 25 °C. A 8.02kg batch of dough was prepared for each trial. All dry raw materials were scaled and added to the spiral mixer bowl. The enzyme solution was added to the bowl with the flour. The preferred amount of enzyme solution to be used will depend on the process used, process conditions, and the ingredients. Yeast and water scaled last to maintain correct temperature. To make the dough, ingredients were mixed in 'Kemper' Spiral mixer with 500 turns/revolutions at slow speed and for 1300 turns/revolutions at fast speed. Enzymes dosages used are summarized in Table 8.

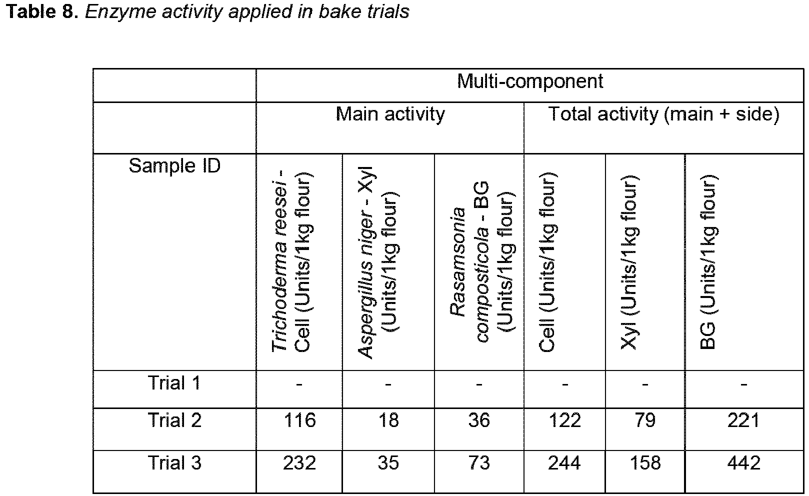

Dough was then removed from the bowl and placed on a floured tray Couche cloth (to prevent skinning) for 10min. Temperature of the dough were recorded at this point.:
Dough was scaled at 800g and moulded round. After 10 minutes rest, the dough was then shaped accordingly. Four of the dough samples were shaped and placed into Loaf tins; the remaining six dough pieces were shaped and placed into floured proving baskets with seam side up. All samples were gently pressed down by hand before entering the proving cabinet. Dough was proved at 38°C and 85% RH (relative humidity) for 40 minutes.

Dough samples in the tin were docked on the surface using the pastry dock and loaded into the ovens. Three of the dough samples in proving baskets were gently tipped out onto the loading belt, docked with pastry dock on the surface and loaded into the oven to be baked on the oven floor. The remaining three samples from the proving baskets were subjected to a 'shock' test in order to assess dough stability. This was achieved by gently tipping the dough out onto a tray lined with baking parchment and docking the surface using the pastry dock. This tray was then dropped from a height of 40cm before loading samples immediately into the oven to be baked on the oven floor.

All trials were baked in Wachtel deck ovens. Top temperature of 235°C, bottom temperature of 270°C with 3 seconds steam injection. Breads were baked for 35min. Trials are then cooled for 2 hours before being packed separately into plastic bags and labelled accordingly.

Table 9 summarises the effect of a MCP on a rye bread formulation containing 20% rye flour sowing an increased basket volume by 5% in the lower dose and 7% in the higher dose. Bread softness was improved by 13% and 15% respectively, one day post baking. All breads possessed a good processability and showed marginal effect on bread shape shown by slight decrease in height to width ratio and a small increase in the width of the breads. Final product characteristics where comparable to control.

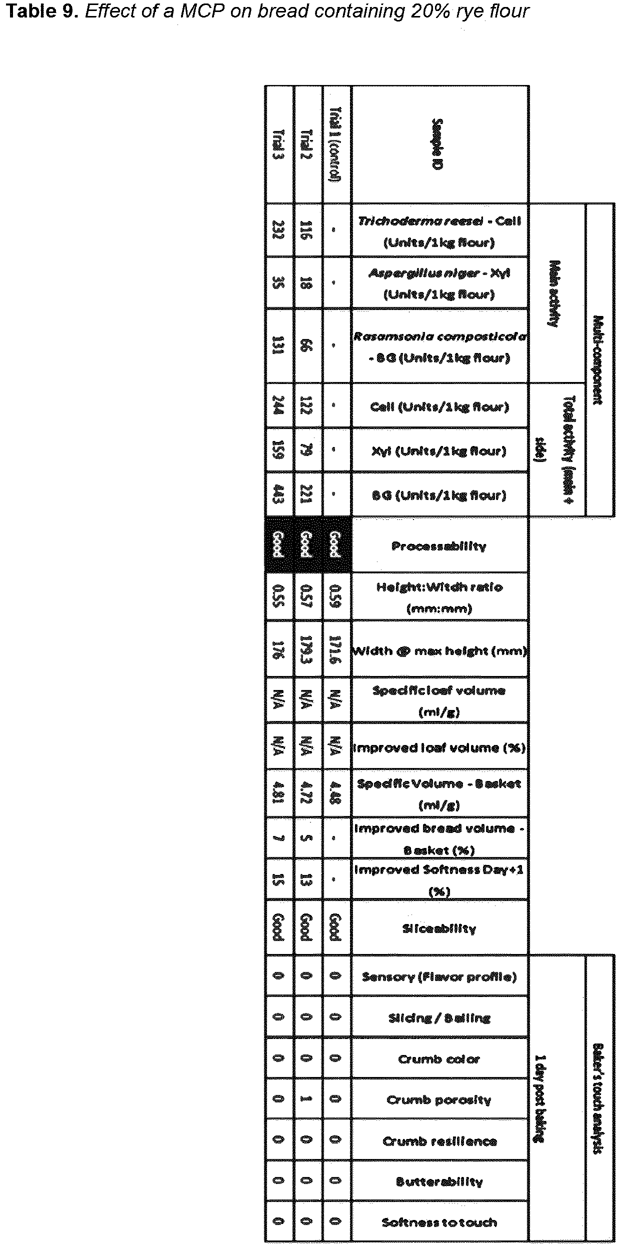

Legend:

| | |
|---|---|
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not acceptable | Very sticky dough, lack of dough stability- machinability and cleaning issues very likely |

### Example 9

### Performance in bread formulation containing whole meal flour

Flour used in the test bakes possessed the following properties:

| | | |
|---|---|---|
| Wheat Flour | Moisture (%) | 15.5 |
| | Protein Content (%) | 11.5 |
| | Ash (%) | 0.60 |
| | Falling Number (sec) | 260 |
| | Farinograph Water Absorption (%) | 59 |
| Whole meal flour | Moisture (%) | 15.5 |
| | Protein Content (%) | 17.4 |
| | Ash (%) | 1.5 |
| | Falling Number (sec) | 280 |
| | Farinograph Water Absorption (%) | 68 |

Composition of the base dough in the test bakes was as follows:

| ***Ingredients*** | **%** | ***Additional Information*** |
|---|---|---|
| Wheat Flour | 40 | Purchased from Meneba, Netherlands (Art.nr. 2821) |
| Whole Meal Flour | 60 | Purchased from Meneba, Netherlands (Art.nr. 2821) |
| Ascorbic Acid | 0.006 | calculated on total flour weight |
| Salt | 1.4 | Standard, calculated on total flour weight |
| Yeast | 3.5 | Compressed Baker's Yeast, calculated on total flour weight |
| Shortening | 1 | Standard, calculated on total flour weight |
| Water | 61 | 25-27°C (calculated by desired dough temperature X2 - 1°C (temperature increase during mixing) - Flour temperature (recorded on the day). 61% calculated on total flour weight |

### Equipment used:

SIGMA Spiral Mixer, 20L Plastic box with lid, 5L plastic bowl, Couche Cloth, 500g Baking Tins (Bread-Loaf), Temperature Probe, pH Probe, Temperature and Humidity controlled proving cabinet, , Wachtel Deck Oven, JAC Bread Slicer, TA Micro Systems, Volscan Profiler, Plastic Zip Lock Bags.

All raw materials being used for the trials came from the same batch code with a good best before date. Flour, calcium propionate, salt and water were all stored at the same ambient temperature. Flour temperature was recorded on each trial day to calculate water temperature in order to achieve desired dough temperature. Water cooled in the fridge was used to achieve the desired water temperature which was lower than room temperature. Desired dough temperature for this Whole meal Bread application is 26-27°C. Desired pH for this Whole meal bread application is 5.8-5.9.

For dough: all dry raw materials were scaled and added to the spiral mixer bowl. The enzyme solution was added to the bowl with the flour. The preferred amount of enzyme solution to be used will depend on the process used, process conditions, and the ingredients. Yeast and water scaled last to maintain correct temperature. To make the dough, ingredients were mixed in 'SIGMA' Spiral mixer for 400 turns/revolutions (slow) and 1800 turns/revolutions (fast). Dough was then removed from the bowl and placed on a floured tray. Temperature and pH of the dough were recorded at this point. Dough firmness and stickiness was evaluated as previously described.

Dough was then covered down with a Couche cloth (to prevent skinning) and left to rest for 10 minutes. Dough was scaled at 820g and moulded round. The dough proofed on the tray for 20 minutes. After the first proving step, the dough pieces were shaped and placed into loaf tins. All samples were gently pressed down by hand before entering the proving cabinet. Dough was proved @32°C and 80% RH (relative humidity) for 60 minutes. Three out of nine prepared samples were subjected to a 'shock' test in order to assess dough stability. This was achieved by dropped from a height of 8 cm tins with dough out onto a table before loading samples immediately into the oven. All trials were baked in Wachtel deck ovens. Top temperature of 260°C, bottom temperature of 230°C. 3 seconds of steam added during the first minute of baking. Trials are then cooled for 2 hours before being packed separately into plastic bags and labelled accordingly.

Study was conducted to assess the technical performance of the MCP solution (trial 2 and 3) against commercial products (trial 4 and 5), which was dosed according the recommended dosage from the supplier.

Table 10a shows the trials containing a MCP perform best by increasing specific bread volume by 11%, followed by a commercial product 2 (10%) and a commercial product 1 (3%). Over a shelf life of seven days, the MCP showed an improved softness ranking from 9% to 21%. The commercial product 2 improved softness by 19% and 15% at day 1 and 7 respectively, while the commercial product 1 only showed a smaller effect on softness of 8%. The improved softness from Trial 3 and 4 was also assessed in the sensorial evaluation, where the softness to touch was higher than negative control. Dough containing commercial product 1 was slightly less extensible and more elastic. Trials 3 and 5 showed also a slight increased in tightness. Trial 3 showed a more open crumb (+2) and improved softness to touch (+2) compared to the control after one day post baking. Both commercial products showed an increased cohesiveness (+2) at day seven post baking.

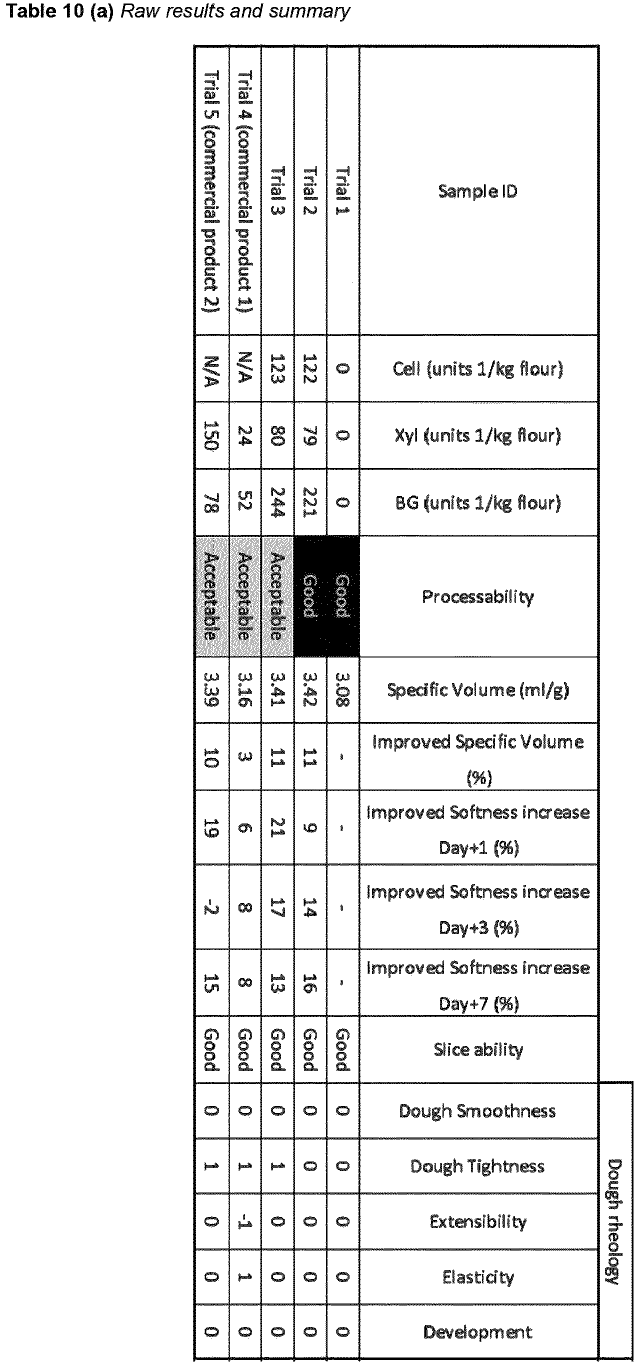

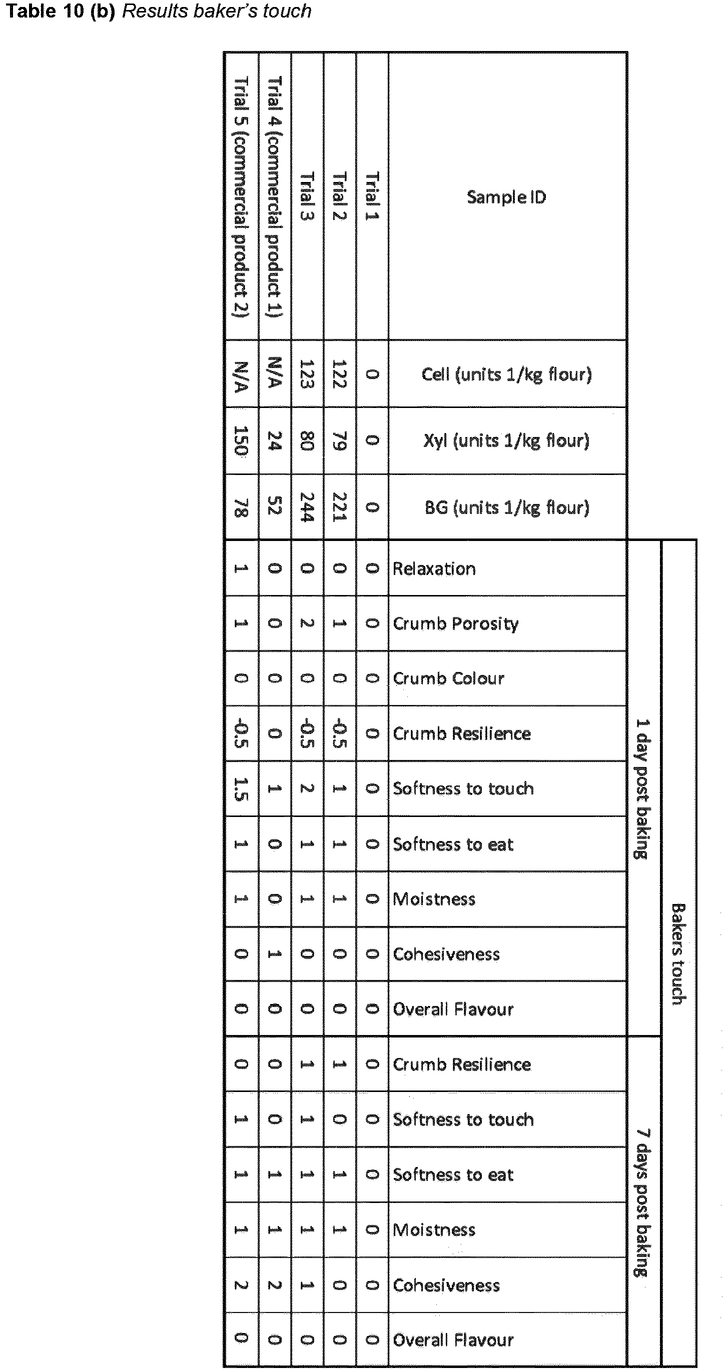

### Example 10

### Bake trials against a commercial product

Bake tests were performed to assess the technical performance of a commercial product against a MCP using a ratio which showed promising results in previous trials. Both enzyme preparations were applied with 3 different dosages in order to investigate the impact on dough rheology and final products characteristics as a result of an increasing dosage. Bake trials were performed following material and method in Example 4 and enzymes dosages were used according Table 11.

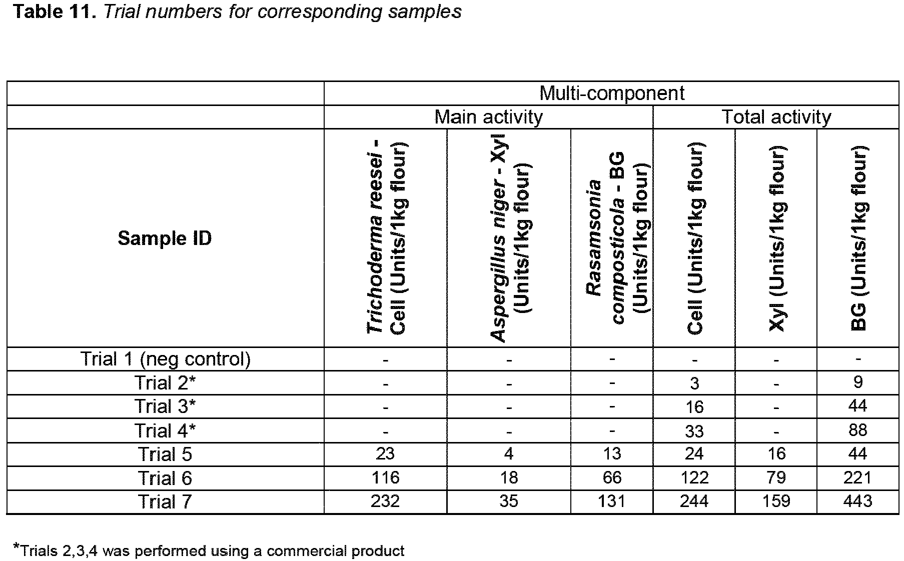

Table 12 summarises the results of final product measurement as well as dough rheology measurements. Using the commercial enzyme, the dough surface was less sticky as a response to an increasing protein content. The dough was also more elastic and less soft (firmer). With an increasing content of a MCP, dough became more sticky and softer, while no difference in elasticity was observed compared to a negative control. The processability of doughs containing the commercial product was considered to be improved with a higher dosage, while an increasing MCP application showed detrimental at the highest dosage indicating a potential overdosing if dosages would be increased further.

In baker's touch analysis after one day post baking, bread containing a MCP were softer to touch while only a higher a -dosage - of the -commercial product resulted in a softer bread crumb. The crumb was also more resilient using a medium and high dosage of a MCP, which is different to commercial product. An improved butterabilty was obtained with the commercial product, which was not evident using a MCP. After 5 days post baking, breads with a MCP were softer to touch, moister and except the higher dosage, did not show any impact on the cohesiveness. On the other hand, the commercial - product showed no difference in softness to touch, were less moist and less cohesive.

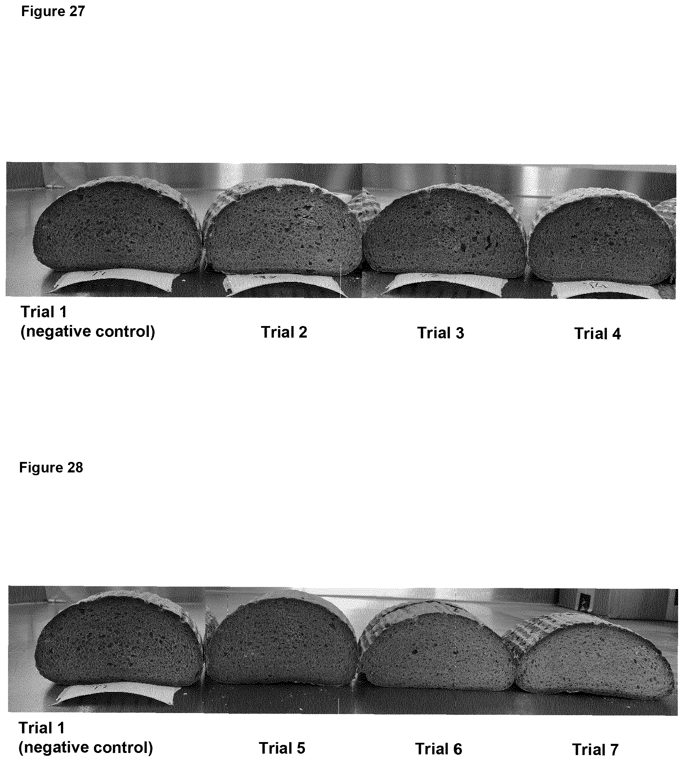

The application of the commercial product shows a stable height to width ratio with an increasing amount of protein per 1 kg flour. Alternatively, the MCP showed a decreasing ratio with an increasing dosage. This is also visible in Figure 27 and Figure 28 showing the differences in the shape of breads of both enzyme preparations. Breads baked with the commercial - products tend to have a more round shape, while a more oval shape was observed using a MCP. Measuring the width to max height support those findings. A MCP showed an increasing width, while the values tend to decrease using the commercial - product.

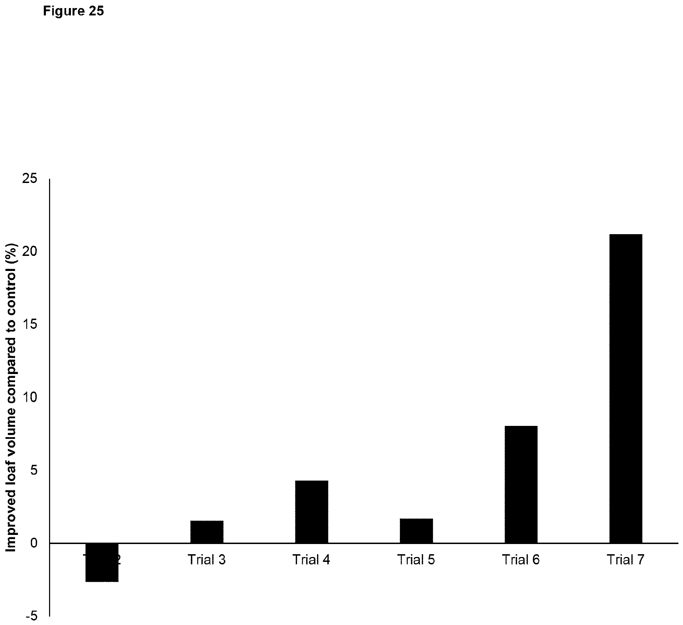

The application of a MCP demonstrates a significant improvement loaf volume from 2% to 21%, while the commercial - enzyme decreased loaf volume by 3% at a low dosage and showed a minor improvement of 4% with highest protein content (Figure 25).

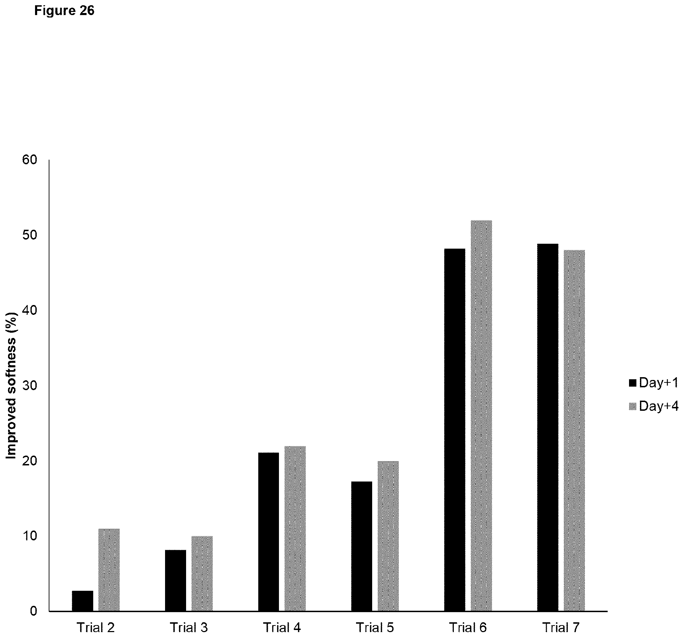

Texture profile analysis on the day 1 correlate with outcomes on day 5 (Figure 26). Loafs containing a MCP showed an improving softness of approximately 20%, 52% and 48% (respective to the low, medium, high dosage) on both days. The commercial product - showed less impact below 12% for low and medium and about 20% with the highest protein content.

The technical performance of both enzyme preparations showed significant differences in rye bread application. Several attributes in the assessment of the dough rheology showed an opposite effect as a response of an increasing dosages. Final product characteristics showed distinct differences. Particular an increase in bread volume and improved bread volume showed little or no benefit using the commercial - - enzyme, but was large effected using a MCP. Opposite outcomes were also obtained when assessing bread shapes as well as height to width ratio of final products.

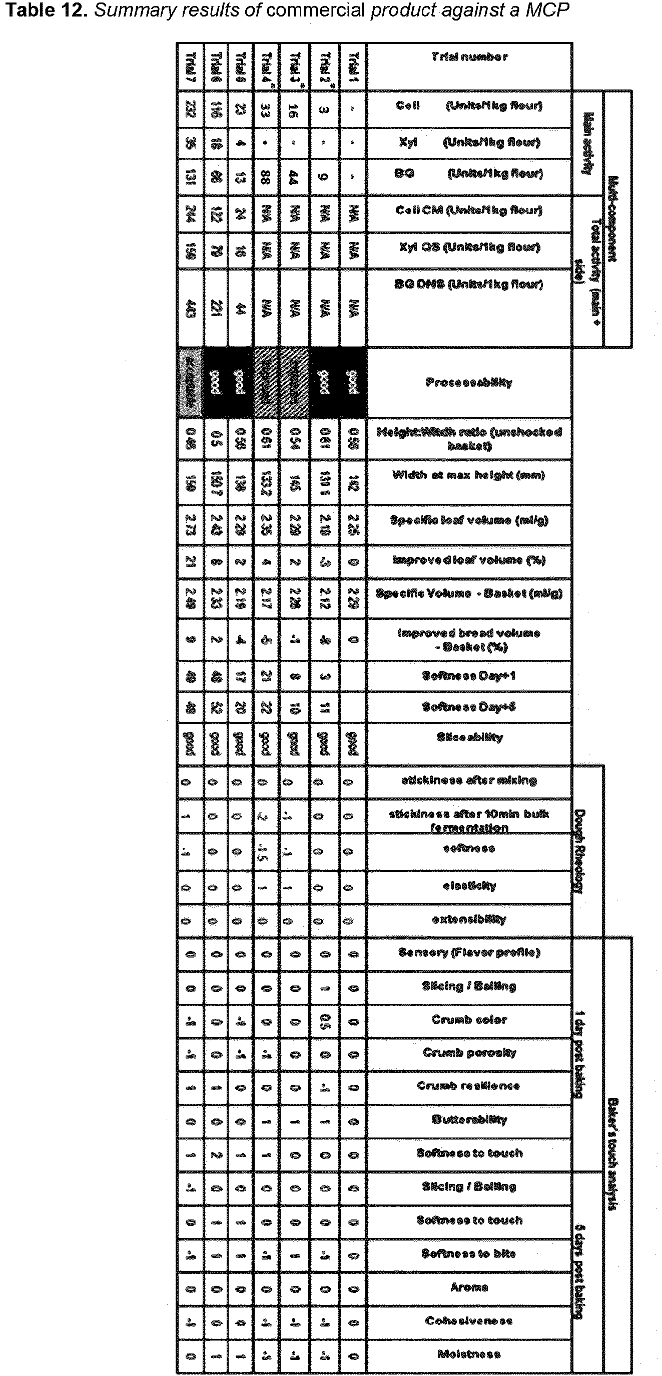

Legend:

| | |
|---|---|
| Improved | Dough shows a decrease in stickiness compared to a reference |
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not acceptable | Very sticky dough, lack of dough stability- machinability and cleaning issues very likely |

### Example 11

### Replacement of a cellulase from A. niger

The performance of a cellulase, produced by A. *niger,* has been tested in rye bread model according of Example 4.

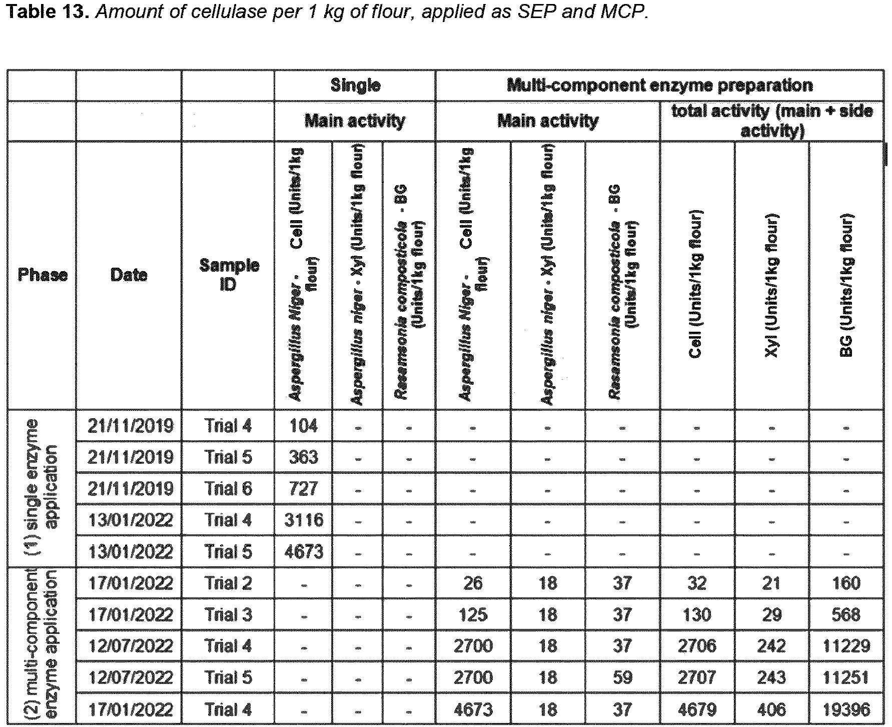

The cellulase from A. *niger* resulted in an improved loaf volume of 3 to 10%, when 104 to 727 Units/1kg flour were applied in a SEP.

In a MCP, 26 to 125 Units/1kg flour improved loaf volume by 13 to 18%.

Breads maintained a good processability, only the highest dosage of 4673 Units/1kg flour had a detrimental effect. Applied in a SEP and MCP, cellulase addition improved softness significantly. Breads also showed a good sliceability and did not cause balling significantly. Crumb characteristics were compared to reference.

Across all breads, no difference in the flavor profile was observed, while breads were more moist 5 days post baking.

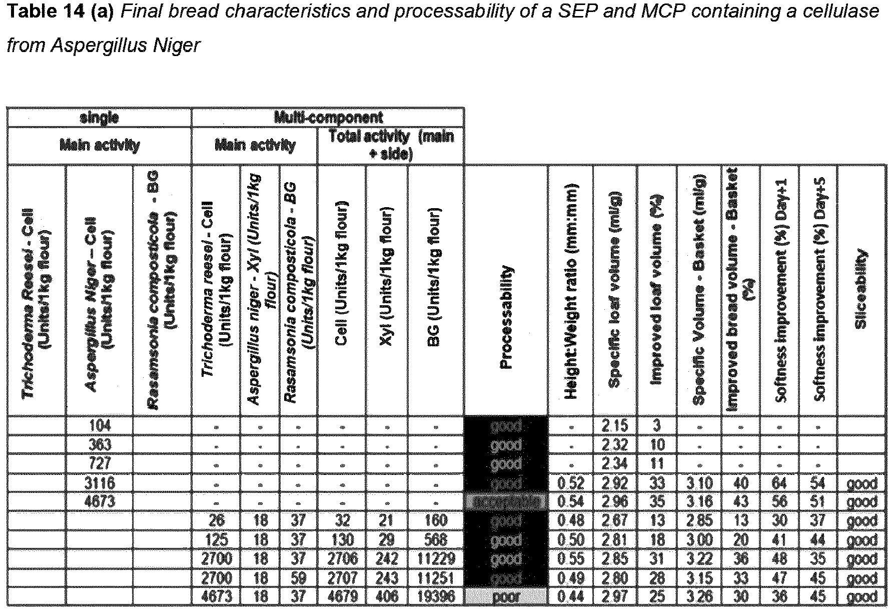

Legend:

| | |
|---|---|
| Good | Minimum of dough stickiness- no issues with machinability or cleaning |
| Acceptable | Slightly sticky, dough stability is acceptable- probably no issue of machinability or cleaning |
| Poor | Sticky dough, lack of dough stability- machinability and cleaning issues probable |
| Not acceptable | Very sticky dough, lack of dough stability- machinability and cleaning issues very likely |

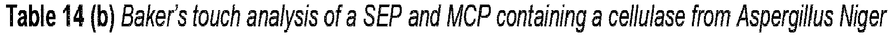

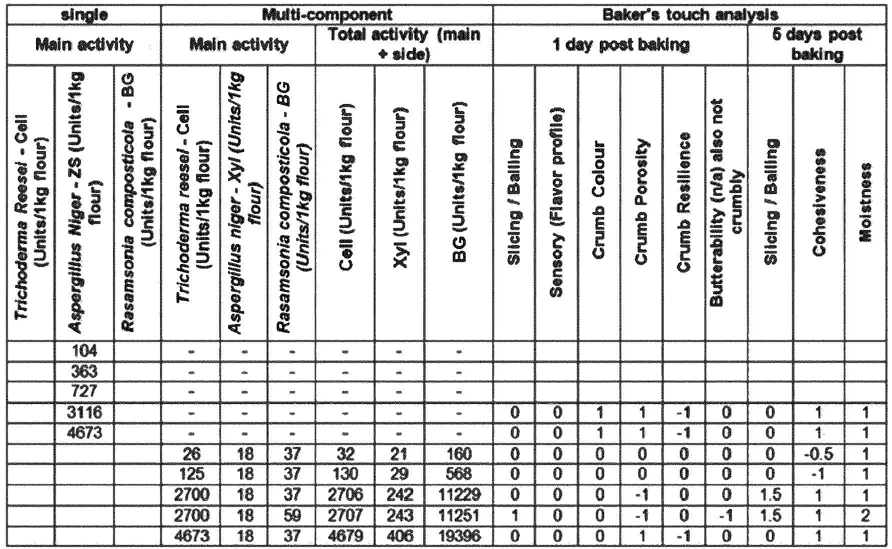

## Claims

1. A dough composition comprising:
(a) flour; and
(b) at least one enzyme selected from a cellulase enzyme, a beta-glucanase enzyme and a xylanase enzyme.

2. The dough composition according to Claim 1, wherein the at least one enzyme selected from a cellulase enzyme, a beta-glucanase enzyme and a xylanase enzyme is derived from any one or more of the genera *Trichoderma, Rasamsonia* and *Aspergillus.*

3. The dough composition according to any one of Claims 1-2, wherein the cellulase enzyme is present in an amount of 29 to 2707 units/kg flour.

4. The dough composition according to any one of Claims 1-3, wherein the cellulase enzyme is derived from the fungus of the species *Trichoderma reesei and*/*or Aspergillus niger and*/*or Rasamsonia composticula .*

5. The dough composition according to any one of Claims 1-4, wherein the cellulase enzyme has at least 70% identity to SEQ ID NO:5; and/or SEQ ID NO:6; and/or SEQ ID NO:7; and/or SEQ ID NO:8; and/or SEQ ID NO:15; and/or SEQ ID NO:16; and/or SEQ ID NO:24; and/or SEQ ID NO:25; and/or SEQ ID NO:26; and/or SEQ ID NO:28; and/or SEQ ID NO:29; and/or SEQ ID NO:30; and/or SEQ ID NO:31; and/or SEQ ID NO:32; and/or SEQ ID NO:33; and/or SEQ ID NO:45; and/or SEQ ID NO:46; and/or SEQ ID NO:47; and/or SEQ ID NO:48; and/or SEQ ID NO:49.

6. The dough composition according to any one of Claims 1-5, wherein the beta-glucanase enzyme is present in an amount of 37 to 11251 units/kg flour.

7. The dough composition according to any one of Claims 1-6, wherein the beta-glucanase enzyme is derived from the fungus of the species *Trichoderma reesei and*/*or Aspergillus niger and*/*or Rasamsonia composticula.*

8. The dough composition according to any one of Claims 1-7, wherein the beta-glucanase enzyme has at least 70% identity to SEQ ID NO:5; and/or SEQ ID NO:6; and/or SEQ ID NO:7; and/or SEQ ID NO:8; and/or SEQ ID NO:15; and/or SEQ ID NO:16; and/or SEQ ID NO:19; and/or SEQ ID NO:20; and/or SEQ ID NO:21; and/or SEQ ID NO:22; and/or SEQ ID NO:24; and/or SEQ ID NO:25; and/or SEQ ID NO:26; and/or SEQ ID NO:28; and/or SEQ ID NO:29; and/or SEQ ID NO:30; and/or SEQ ID NO:31; and/or SEQ ID NO:32; and/or SEQ ID NO:33; and/or SEQ ID NO:39; and/or SEQ ID NO:40; and/or SEQ ID NO:41; and/or SEQ ID NO:44; and/or SEQ ID NO:45; and/or SEQ ID NO:46; and/or SEQ ID NO:47; and/or SEQ ID NO:48; and/or SEQ ID NO:49

9. The dough composition according to any one of Claims 1-8, wherein the xylanase enzyme is present in an amount of 18 to 243 units/kg flour.

10. The dough composition according to any one of Claims 1-9, wherein the xylanase enzyme is derived from the fungus of the species *Trichoderma reesei and*/*or Aspergillus niger and*/*or Rasamsonia composticula.*

11. The dough composition according to any one of Claims 1-10, wherein the xylanase enzyme has at least 70% identity to SEQ ID NO:1; and/or SEQ ID NO:2; and/or SEQ ID NO:3; and/or SEQ ID NO:4; and/or SEQ ID NO:10; and/or SEQ ID NO:11; and/or SEQ ID NO:12; and/or SEQ ID NO:13; and/or SEQ ID NO:14; and/or SEQ ID NO:27; and/or SEQ ID NO:34; and/or SEQ ID NO:35; and/or SEQ ID NO:36; and/or SEQ ID NO:37; and/or SEQ ID NO:38.

12. The dough composition according to any one of Claims 1-11, wherein the dough composition comprises a cellulase enzyme, a xylanase enzyme and a beta-glucanase enzyme.

13. A method of preparing a baked food product; the method comprising:
(a) preparing a dough composition according to any one of Claims 1-12; and
(b) baking the dough for a time and temperature sufficient to yield the bakery product.

14. A method of preparing a baked food product having at least one improved property; the method comprising:
(a) preparing a dough composition according to any one of Claims 1-12; and
(b) baking the dough for a time and temperature sufficient to yield the bakery product;
wherein the at least one improved property is selected from any one or more of volume (specific volume), softness, butterability and balling after slicing, moisture, crumb resilience.

15. Use of the dough composition according to any one of Claims 1-12 in baked foods.
